(19) Europäisches Patentamt
European Patent Office
Office européen des brevets

(11) Publication number: **0 081 321**
**A1**

# (12) EUROPEAN PATENT APPLICATION

(21) Application number: 82306277.3

(22) Date of filing: 25.11.82

(51) Int. Cl.³: C 07 C 101/72
C 07 C 149/43, A 61 K 31/215

(30) Priority: 25.11.81 JP 187730/81

(43) Date of publication of application:
15.06.83 Bulletin 83/24

(84) Designated Contracting States:
AT BE CH DE FR GB IT LI LU NL SE

(71) Applicant: ONO PHARMACEUTICAL CO., LTD.
No. 14, Doshomachi 2-chome Higashi-ku
Osaka-shi Osaka(JP)

(72) Inventor: Miyamoto, Tsumoru
1-40, Koaza Moriyama
Aza Tono Johyoh-shi Kyoto(JP)

(72) Inventor: Mohri, Tetsuya
No 14-3, Ohsumigaoka 4-chome
Tanabe-cho Tsuzuki-gun Kyoto(JP)

(72) Inventor: Shimoji, Katsuichi
No 73-17, Tsunoe 1-chome
Takatsuki-shi Osaka(JP)

(72) Inventor: Wakatsuka, Hirohisa
3-3-708, Miyono-cho
Takatsuki-shi Osaka(JP)

(72) Inventor: Itoh, Hiroyuki
16-20 Yamate-dai 3-chome
Ibaraki-shi Osaka(JP)

(72) Inventor: Hayashi, Masaki
5-10 Nanpei-dai 4-chome
Takatsuki-shi Osaka(JP)

(72) Inventor: Hashimoto, Shinsuke
2-6-905, Shirakawa 3-chome
Ibaraki-shi Osaka(JP)

(74) Representative: Pearce, Anthony Richmond et al,
Marks & Clerk Alpha Tower Suffolk Street Queensway
Birmingham B1 1TT(GB)

## (54) 2-Aminophenol derivatives.

(57) 2-AMINOPHENOL derivatives of the general formula:

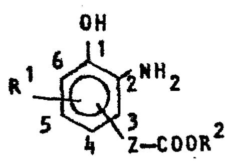

(1)

[wherein Z represents a straight alkylene or alkenylene group containing from 1 to 6 carbon atom(s), $R^1$ represents a hydrogen atom, a halogen atom, a straight or branched chain alkyl group containing from 1 to 6 carbon atom(s), a straight or branched chain alkoxy group containing from 1 to 4 carbon atom(s) or a group represented by general formula:

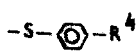

(wherein $R^4$ represents a hydrogen atom or a straight or branched chain alkyl group containing from 1 to 6 carbon atom(s).), $R^2$ represents a hydrogen atom, a straight or branched chain alkyl group containing from 1 to 4 carbon atom(s) or a group represented by a formula:

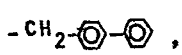

$R^1$ is attached to the 6th or 4th position of the benzene ring: when $R^1$ is attached to the 6th position of the benzene ring, Z-COOR$^2$ is attached to the 4th position of the benzene ring, and when $R^1$ is attached to the 4th position of the benzene ring, Z-COOR$^2$ is attached to the 6th position of the benzene ring.]
and pharmaceutically acceptable acid addition salts thereof, possess a strong inhibitory effect on 5-lipoxygenase specifically, or 5-lipoxygenase and cyclooxygenase at the same time, and therefore, are useful as treating and preventing agents for diseases which induced by leukotrienes, e.g. allergic tracheal and bronchial diseases or allergic lung diseases, allergic shocks or various allergic inflammations, and various inflammations induced by prostaglandins.

EP 0 081 321 A1

SPECIFICATION

2-Aminophenol derivatives

This invention is concerned to new 2-aminophenol derivatives which have an inhibitory activity on 5-lipoxygenase specifically, or an inhibitory activity on 5-lipoxygenase and cyclooxygenase at the same time, a process for their preparation and pharmaceutical compositions containing them or it as an active ingredient.

Up to now, several compounds which inhibit lipoxygenase have been known, that is to say, 5,8,11,14-eicosatetraynoic acid (i.e. EYTA), 5,8,11-eicosatriynoic acid, acetone phenylhydrazone (i.e. APH), phenidone (i.e. 1-phenyl-3-pyrazolidone), NDGA (nordihydroguaiaretic acid), BW-775C (3-amino-1-p-trifluoromethyl-pyrazole), 3-amino-1-p-chlorophenyl-2-pyrazoline, benoxaprofene.

These above compounds inhibit all lipoxygenases and other enzymes in the arachidonate cascade at the same time, or inhibit all lipoxygenases of 5-, 12- and 15-lipoxygenase [see Gendai Iryo , 12, 1065 (1980)].

The compounds of the present invention are particularly superior in having selective inhibitory activity on 5-lipoxygenase of many lipoxygenases,which react at the first stage of producing various kinds of leukotrienes from arachidonic acid in a living body and further not inhibiting other enzymes in the arachidonate cascade, or in having inhibitory activity on 5-lipoxygenase and cyclooxygenase which reacts at the first step of producing prostaglandins from arachidonic acid similarly, at the same time.

This invention is concerned to new 2-aminophenol derivatives which have an inhibitory activity on 5-lipoxygenase, or an inhibitory activity on 5-lipoxygenase and on cyclooxygenase at the same time, the process for their preparation and pharmaceutical compositions containing them as an active ingredient. More particularly, there are a group of compounds called leukotrienes, which are biosynthesized from arachidonic acid by a series of reactions in a living body and 5-lipoxygenase is related to the first step of this reaction. Similarly, prostaglandins are biosynthesized from arachidonic acid by another series of reactions, and cyclooxygenase is related to the first step of such reaction. This invention is concerned to new 2-aminophenol derivatives which strongly inhibit 5-lipoxygenase or strongly inhibit 5-lipoxygenase and cyclooxygenase at the same time, and which are, therefore, useful as treating and preventing agents for diseases which induced by leukotrienes, e.g. allergic tracheal and bronchial diseases or allergic lung diseases, allergic shocks or various allergic inflammations, and various inflammations induced by prostaglandins, and further, this invention is concerned to the process for their preparation and pharmaceutical compositions comprising them as an active ingredient.

In the study of prostaglandins (abbreviated as PG hereafter), many important discoveries have been made continuously in recent years. And so it was found a large change in the direction of the research and development of PG. In the compounds which have been newly found or newly confirmed their structure in PG family, it can be said that PG endoperoxides, (i.e. $PGG_2$ and $PGH_2$), thromboxane $A_2$ (abbreviated as $TXA_2$ hereafter), prostacyclin (i.e. $PGI_2$) and leukotriene C, D and E

(abbreviated as LTC, LTD and LTE, respectively, hereafter) etc. have especially strong and unique biological activities.

All the compounds of PG family containing various PG previously known well in addition to the above compounds, are biosynthesized from the same mother compound, i.e. arachidonic acid in a living body and, therefore, the metabolic routes starting from arachidonic acid is called "Arachidonate cascade" as a whole. The detailed explanation of each route and the pharmacological character of each metabolite are described in Igaku no Ayumi, 114, 378 (1980), ibid, 114, 462 (1980), ibid, 114, 866 (1980), ibid, 114, 929 (1980), Gendai Iryo, 12, 909 (1980), ibid, 12, 1029 (1980), ibid, 12, 1065 (1980) and ibid, 12, 1105 (1980) etc.

The arachidonate cascade can be largely divided into two routes; one is the route that cyclooxygenase acts on arachidonic acid to convert, via $PGG_2$ and further $PGH_2$, into various PG, e.g. prostaglandin $F_{2\alpha}$ (abbreviated $PGF_{2\alpha}$ hereafter), prostaglandin $E_2$ (abbreviated $PGE_2$ hereafter), $PGI_2$, $TXA_2$, and the other is the route that lipoxygenase acts on arachidonic acid to convert, via hydroperoxyeicosatetraenoic acid (abbreviated HPETE hereafter), into hydroxyeicosatetraenoic acid (abbreviated as HETE hereafter) or leukotrienes.

As the former route is well known, it is not described in the present specification in detail. See Prostaglandin (1978), edited by Makoto Katori, published by Kohdan-sha.

Concerning the latter route, it has been known that various compounds are produced according to the following Scheme A.

4'

SCHEME A

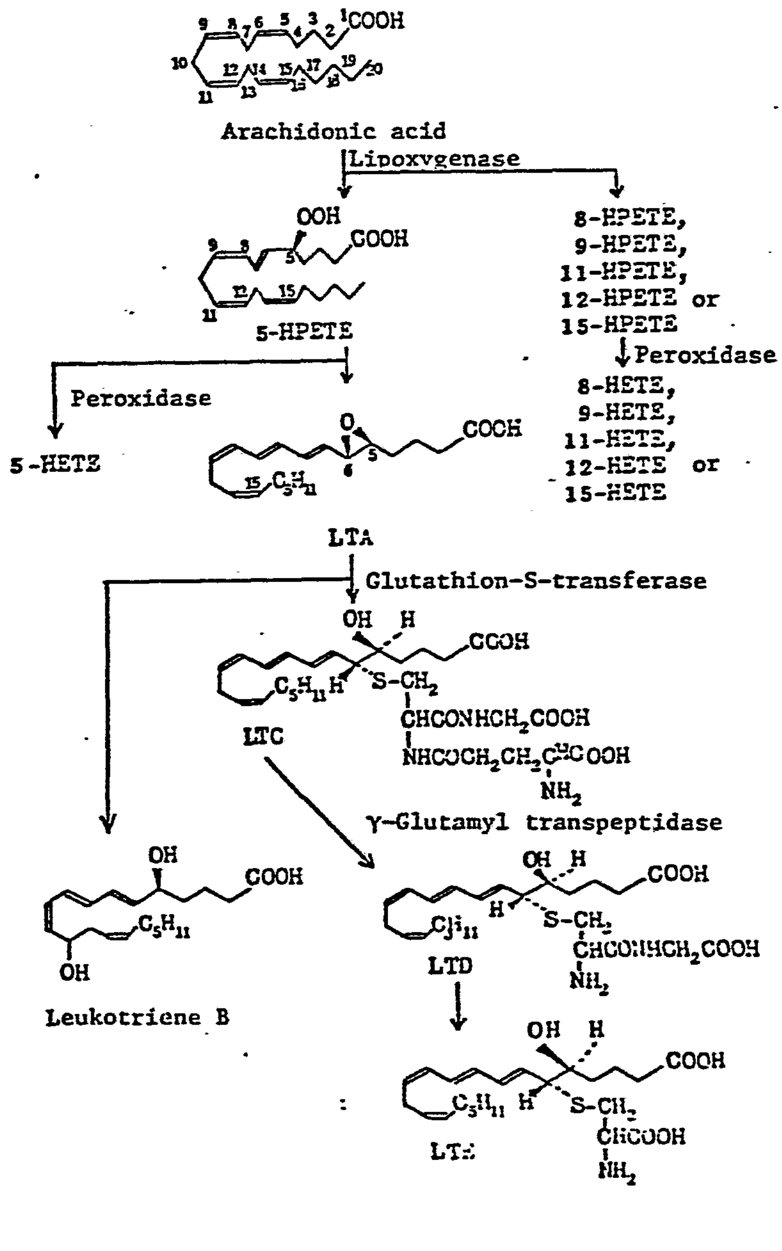
Arachidonic acid
Lipoxygenase
5-HPETE
8-HPETE,
9-HPETE,
11-HPETE,
12-HPETE or
15-HPETE
Peroxidase
8-HETE,
9-HETE,
11-HETE,
12-HETE or
15-HETE
Peroxidase
5-HETE
LTA
Glutathion-S-transferase
LTC
γ-Glutamyl transpeptidase
Leukotriene B
LTD
LTE

Besides being metabolized through a well known route, i.e. the route via PG endoperoxides, arachidonic acid is also metabolized through another route by the action of lipoxygenase. That is to say, arachidonic acid is metabolized by the action of lipoxygenase, e.g. 5-lipoxygenase, 12-lipoxygenase and 15-lipoxygenase, to 5-HPETE, 12-HPETE and 15-HPETE, respectively, having following formulae:

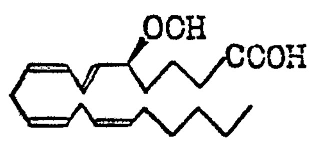

,

5-HPETE

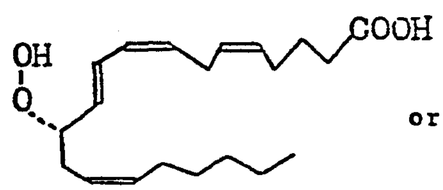

or

12-HPETE

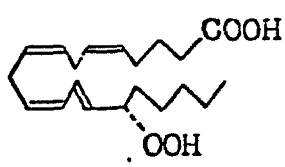

15-HPETE

These HPETE are converted into 5-HETE, 12-HETE and 15-HETE, respectively, by the action of peroxidase converting a hydroperoxy group into a hydroxy group. Furthermore, LTA is also produced from 5-HPETE. LTA is converted into leukotriene B (abbreviated as LTB hereafter) enzymatically or not enzymatically, or is converted into LTC by the action of glutathion-S-transferase. Further, LTC is converted into LTD by the action of γ-glutamyl transpeptidase.

Moreover, it was recently defined that LTD is metabolized to LTE [see Biochem. Biophys. Res. Commun., 91, 1266 (1979) and Prostaglandins, 19(5), 645 (1980)].

It was found that LTC and LTD are identical with SRS (slow reacting substance) or SRS-A (slow reacting substance of anaphylaxis) which was well known before [see Proc. Natl. Acad. Sci. USA, 76, 4275 (1979), Biochem. Biophys. Res. Commun., 91, 1266 (1979), Proc. Natl. Acad. Sci. USA, 77, 2014 (1980) and Nature, 285, 104 (1980)]. So it can be understood that the pharmacological characters of these leukotrienes are the same as those of SRS or SRS-A.

Feldberg et al. reported that a substance is released when perfusing cobra venom through isolated lung or incubating cobra venom with vitellus. The substance was named SRS and it has been found that SRS constricts ilem isolated from guinea pig slowly and continually [see J. Physiol., 94, 187 (1938)].

Moreover, Kellaway et al. showed the relation between SRS-A and allergic reaction from the fact that SRS-A is released when an antigen is sensitized to perfusing lung isolated from guinea pig [see Quant. J. Exp. Physiol., 30, 121 (1940)].

Brocklehurst reported that when the antigen is sensitized to a

lung fragment isolated from a bronchial asthmatics whose specific antigen is defined, by an operation, histamine and SRS-A are released and strongly constrict bronchial muscle. Since such constriction can not be prevented by an antihistaminic agent, he suggested that SRS-A is an important bronchoconstrictor in an asthmatic paroxysm [see Progr. Allergy, 6, 539 (1962)].

Since then, many reports were published, for instance, SRS-A prepared from human lung slice constrict a tracheal spiral of normal human [see Int. Arch. Allergy Appl. Immunol., 38, 217 (1970)]; when SRS-A prepared from rats is injected intravenously to guinea pig, significant increase of purlmonory resistance is observed [see J. Clin. Invest., 53, 1679 (1974)]; in addition, a subcutaneous injection of SRS-A to guinea pig, rat and monkey makes vascular permeability to enhance. [see Advances in Immunology, 10, 105 (1969), J. Allergy Clin. Immunol., 621, 371 (1978), Prostaglandins, 19(5), 779 (1980) etc.].

Generally, the substance released by immunological reaction is called SRS-A. On the other hand, the substance released by non-immunological reaction such as calcium ionophore is called SRS. However, the above two substances have many similarities each other and, therefore, it is considered they would probably be the same substance.

Based on result of these studies, various leukotrienes (LTC, LTD and LTE, and further other leukotrienes which may be confirmed their structures in future) biosynthesized from arachidonic acid via LTA, are considered to be important factors relating to the appearance of allergic tracheal and bronchial disease, allergic lung disease, allergic shock and various allergic inflammations.

Recently, it was proposed that PG is an important chemical

mediator of inflammatory reaction. For example, it was reported that PGE enhances a vascular permeability and a pain, and has a vasodilative action, pyrogenetic action and chemotactic action [see Prostaglandin (1978), edited by Makoto Katori, published by Kohdan-sha]. Furthermore, it was reported that $PGI_2$ alone does not affected vascular permeability, but it enhances vascular permeability of histamine [see Prostaglandins, 15, 557 (1978)]. Moreover, the most recent report indicated that in order to suppress such action of PG, it is more effective to inhibit not only 5-lipoxygenase but also cyclooxygenase at the same time [see Biochemical Pharmacology, 28, 1959 (1979) and European Journal of Pharmacology, 66, 81 (1980)].

As the result of energetic investigations in order to find new compounds inhibiting 5-lipoxygenase, or inhibiting 5-lipoxygenase and cyclooxygenase at the same time in a living body, and so being effective for prevention and treatment of not only various allergic diseases induced by leukotrienes but also inflammations induced by PGs, we have found the compounds which can be achieved that purpose, having completed this invention.

That is to say, the present invention is concerned with 2-aminophenol derivatives of the general formula:

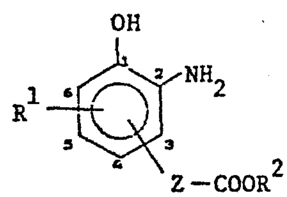

(I)

[wherein Z represents a straight alkylene or alkenylene group containing from 1 to 6 carbon atom(s), $R^1$ represents a hydrogen atom, a halogen atom, a straight or branched chain alkyl group containing from 1 to 6 carbon atom(s), a straight or branched chain alkoxy group containing from 1 to 4 carbon atom(s) or a group represented by general formula: 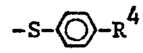 (wherein $R^4$ represents a hydrogen atom or a straight or branched chain alkyl group containing from 1 to 6 carbon atom(s).), $R^2$ represents a hydrogen atom, a straight or branched chain alkyl group containing from 1 to 4 carbon atom(s) or a group represented by a formula: 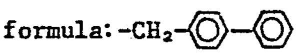 , $R^1$ is attached to the 6th or 4th position of the benzene ring; when $R^1$ is attached to the 6th position of the benzene ring, Z-$COOR^2$ is attached to the 4th position of the benzene ring, and when $R^1$ is attached to the 4th position of the benzene ring, Z-$COOR^2$ is attached to the 6th position of the benzene ring.]

or pharmaceutically acceptable acid addition salts thereof.

The compounds of the general formula (I) include compounds of the general formula:

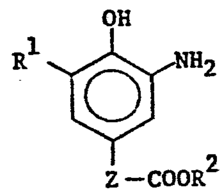

(Ia)

[wherein all of the symbols represent the same meanings as hereinbefore defined.]

and compounds of general formula:

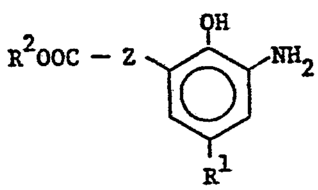

(Ib)

[wherein all of the symbols represent the same meanings as hereinbefore defined.].

In the general formula (I), (Ia) and (Ib), examples of the straight alkylene or alkenylene group represented by Z are methylene, ethylene, trimethylene, tetramethylene, pentamethylene and hexamethylene groups, and vinylene, n-propenylene, n-butenylene, butadienylene, n-pentenylene, pentadienylene, n-hexenylene, n-hexadienylene and n-hexatrienylene groups. Preferable groups are methylene, ethylene, vinylene, n-propenylene and butadienylene groups.

In the general formula (I), (Ia) and (Ib), in halogen atoms represented by $R^1$, preferable atoms are chlorine and fluorine atoms.

In the general formula (I), (Ia) and (Ib), examples of the alkyl group containing from 1 to 6 carbon atom(s) represented by $R^1$ are methyl, ethyl, propyl, butyl, pentyl and hexyl groups, and isomers thereof. Preferable group is t-butyl group.

In the general formula (I), (Ia) and (Ib), examples of the alkoxy group containing from 1 to 4 carbon atom(s) represented by $R^1$ are methoxy, ethoxy, propoxy and butoxy groups, and isomers thereof. Preferable group is a methoxy group.

In the general formula (I), (Ia) and (Ib), examples of the alkyl group containing from 1 to 4 carbon atom(s) represented by $R^2$ are methyl, ethyl, propyl and butyl groups, and isomers thereof. Preferable groups are methyl and ethyl groups.

In the compounds.of the general formula (I), (Ia) and (Ib), compounds wherein $R^2$ represents a hydrogen atom or the group represented by the formula: $-CH_2-$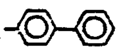 are also preferable.

In the general formula (I), (Ia) and (Ib), it is also preferable the case that $R^1$ represents the general formula: -S-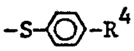-$R^4$ (wherein examples of the straight or branched chain alkyl group containing from 1 to 6 carbon atom(s) represented by $R^4$ are methyl, ethyl, propyl, butyl, pentyl and hexyl groups and isomers thereof. And preferable groups represented by $R^4$ are a hydrogen atom and a n-pentyl group).

In the compounds of the general formula (I), (Ia) and (Ib), preferable compounds are compounds wherein Z represents a methylene, ethylene, vinylene, n-propenylene or butadienylene group, and $R^1$ represents a hydrogen or chlorine atom, or a methoxy group or a group of the general formula: -S-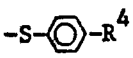-$R^4$ (wherein preferable groups represented by $R^4$ are a hydrogen atom or a n-pentyl group), and $R^2$ represents a hydrogen atom or methyl or ethyl group or a group represented by the formula: $-CH_2-$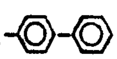 .

In the compounds of the general formula (I), (Ia) and (Ib), when Z represents an alkenylene group, two isomers in <u>trans</u> and <u>cis</u> configuration may naturally exist by each double bond. However, the

present invention include each isomers and the mixture thereof.

Recently, a patent application including a part of compounds of the present application, i.e. compounds wherein $R^1$ is a hydrogen atom and $R^2$ is a carboxyl group in the general formula (I), has been published [see the European Patent Publication No. 0031561A2]. That is, the published specification is concerned to compounds of the general formula:

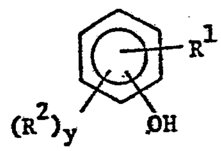

[wherein $R^1$ represents $-COOH$, $-(CH_2)nCOOH$, $-CH=CH-CH=CH-COOH$, $-CH(R^3)COOH$, $-O-(CH_2)n-COOH$, $-S-(CH_2)n-COOH$ etc., n represents zero or an integer of from 1 to 5, $R^3$ represents an alkyl group containing from 1 to 6 carbon atom(s) or a hydroxy group, $R^2$ represents a hydroxy group, a lower alkoxy group containing from 1 to 10 carbon atom(s), a lower carbonylalkoxy group, an amino group etc. and y represents an integer of 1 or 2 (the definitions of such symbols are restricted to ones relative to the present invention.)].

However, the above application may not be considered to be a prior art of our present invention, because it is obscure whether a compound containing an amino group is synthesized in fact and because the use of such compounds is different from that of the present invention, taking into consideration the following facts:

(1) it is described that the only use of the compounds of the published specification is adjuvants for pharmaceuticals and it is not described that the compounds have the same bioactivity, i.e. inhibitory activity on 5-lipoxygenase selectively or on 5-lipoxygenase

and cyclooxygenase at the same time as the compounds of the present invention possess;

(2) the concrete methods for the preparation of the compounds are not disclosed in the specification, and further there is no quotation literature for the preparation; and

(3) in preparing Examples, there is no description of compounds including amino group(s) which is an essential group of our present invention.

Therefore, the present invention includes the above compounds.

Furthermore, in compounds of the present invention, 2-amino-4-carboxymethylphenol and their ester, i.e. 2-amino-4-methoxycarbonylmethylphenol are described in the Japanese Patent Kokai No. 50-70361. In this specification, it is described that the above compounds may be used as intermediates for bicycloheterocyclic compounds having pharmacological activities, i.e. analgesic, antipyretic and antiinflammatory activities, but there are no description about bioactivities, i.e. inhibitory activity on 5-lipoxygenase or on 5-lipoxygenase and cyclooxygenase at the same time.

Therefore, the present invention includes the new use of the above compounds.

The compounds of the general formula (I) are largely divided into two groups in the difference of Z. That is, one group is compounds of general formula:

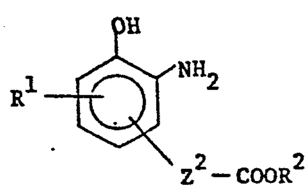

(Ic)

[wherein $Z^2$ represents a straight alkenylene group containing from 2 to 6 carbon atoms, $R^1$ and $R^2$ represent the same meaning as hereinbefore

defined, and the attached positions of $R^1$ and $Z^2$-$COOR^2$ apply correspondingly as the ones of $R^1$ and Z-$COOR^2$ hereinbefore depicted].

and the other group is compounds of general formula:

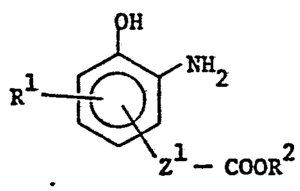

(Id)

[wherein $Z^1$ represents a straight alkylene group containing from 1 to 6 carbon atom(s), $R^1$ and $R^2$ represent the same meaning as hereinbefore defined, and the attached positions of $R^1$ and Z-$COOR^2$ apply correspondingly as the ones of $R^1$ and Z-$COOR^2$ hereinbefore depicted.]

According to a feature of the present invention, compounds of the general formula (Ic) may be prepared by selective reduction to an amino group, of the nitro group of a compound of general formula:

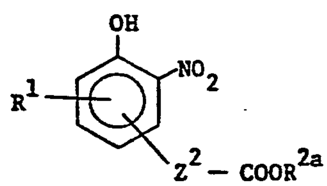

(IIa)

[wherein $R^{2a}$ represents a hydrogen atom or a straight or branched chain alkyl group containing from 1 to 4 carbon atom(s), and the other symbols represent the same meaning as hereinbefore defined.] or a compound of general formula:

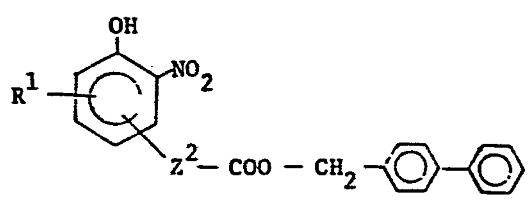

(IIc)

[wherein all of the symbols represent the same meaning as hereinbefore defined]

Compounds of the general formula (IIc) may be prepared by esterification of a compound of the general formula (IIa) wherein $R^{2a}$ represents a hydrogen atom, with 4-biphenylmethanol.

Esterification may be carried out, for example, by using a condensing reagent such as p-toluenesulfonyl chloride in an amine such as pyridine as a solvent, with molecular sieves with refluxing.

The reduction may be carried out, for example, with using iron, zinc, tin or stannous chloride in hydrochloric acid, with using titanium trichloride in an inert organic solvent such as benzene or tetrahydrofuran, with using sodium persulfide or ammonium sulfide in an aqueous alcohol, or with using sodium hydrofulfite in ammonia water (this method is preferable); and the above methods are abbreviated as reduction method A.

Compounds of the general formula (Id) with the exception of compounds wherein $Z^1$ represents a methylene group, may be prepared by reduction to an amino group, of the nitro group and to a saturated alkylene group, of the double bond(s) in $Z^2$ of the compounds of the general formula (IIa) at the same time.

The reduction may be carried out, for example, with using hydrogen gas, by catalytic reduction, in the presence of catalyst such as nickel, platinum, palladium-carbon, palladium or copper-cromium oxide in an inert organic solvent (this method is preferable) or with using diboran or aluminum hydride; and the above methods are abbreviated as reduction method B.

In compounds of the general formula (Id), compounds wherein $Z^1$ represents a methylene group may be prepared by reduction to an amino group, of the nitro group of compounds of general formula:

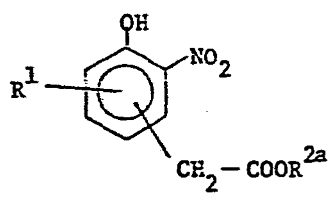

(IIb)

[wherein $R^1$ represents the same meaning as hereinbefore defined, and the attached positions of $R^1$ and $CH_2$-$COOR^{2a}$ apply correspondingly as the ones of $R^1$ and Z-$COOR^2$ hereinbefore depicted].

The reduction may be carried out, by the reduction method A or B mentioned above.

In compounds of the general formula (Id), compounds wherein $R^2$ represents 4-biphenylmethyl group may be prepared by esterification of compounds of the general formula (Id) wherein $R^2$ represents a hydrogen atom.

Esterification may be carried out by methods known per se, for example, the same method which is used in preparation of the compounds of the general formula (IIc) from the compounds of the general formula (IIa).

Compounds of the general formula (IIb) may be prepared by nitration of a compound of general formula:

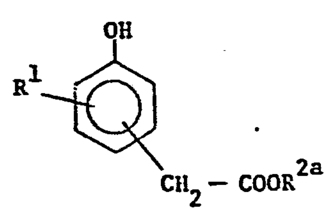

(III)

[wherein all of the symbols represent the same meaning as hereinbefore defined]

The nitration may be carried out, with using a conventional nitrating reagent such as mixed acid of nitric acid and sulfuric acid, a mixture of nitric acid and sulfuric acid in acetic acid or nitric acid in acetic acid (this reagent is preferable) etc.

Compounds of the general formula (III) are known per se or may be prepared by known methods. For example, p-hydroxyphenylacetic acid is available as a commercial product.

Compounds of the general formula (IIa) may be prepared;

(1) by nitration, by the foregoing method, of a compound of general formula:

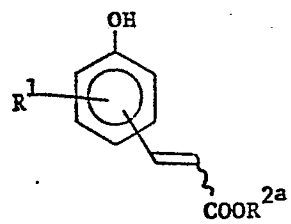

(IV)

[wherein $R^1$ and $R^{2a}$ represent the same meaning as hereinbefore defined, '~~' represents that $COOR^{2a}$ may be attached in the cis side or trans side of the double bond, and the attached positions of $R^1$ and ⁄⁀~$COOR^{2a}$ apply correspondingly as the ones of $R^1$ and Z-$COOR^2$ hereinbefore depicted]

or (2) by Wittig reaction of a compound of general formula:

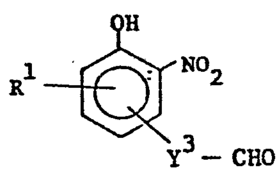

(V)

[wherein $Y^3$ represents a direct bond or a methylene group, $R^1$ represents

the same meaning as hereinbefore defined, and the attached positions of $R^1$ and $Y^3$-CHO apply correspondingly as the ones of $R^1$ and Z-$COOR^2$ hereinbefore depicted]

and a phosphorane compound of general formula:

$$Ph_3P = CH - X^2 - COOR^3 \qquad \text{(VIa)}$$

[wherein Ph represents a phenyl group, $X^2$ represents a direct bond or a straight alkenylene group containing from 2 to 4 carbon atoms, and $R^3$ represents a straight or branched chain alkyl group containing from 1 to 4 carbon atom(s)]

or a phosphonium salt of general formula:

$$Ph_3P^{+} - CH_2 - X^1 - COOH \cdot Br^{-} \qquad \text{(VIIa)}$$

[wherein $X^1$ represents a straight alkylene group containing from 1 to 4 carbon atom(s), and Ph represents the same meaning as hereinbefore defined] or

(3) by Wittig reaction of a compound of general formula:

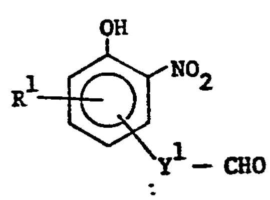

(IX)

[wherein $Y^1$ represents a straight alkylene group containing from 2 to 4 carbon atoms, $R^1$ represents the same meaning as hereinbefore defined, and the attached positions of $R^1$ and $Y^1$-CHO apply correspondingly as the ones of $R^1$ and Z-$COOR^2$ hereinbefore depicted]

and a phosphorane compound of the general formula:

$$Ph_3P=CH-X^4-COOR^3 \qquad (VIb)$$

[wherein $X^4$ represents a direct bond or a vinylene group, and Ph and $R^3$ represent the same meaning as hereinbefore defined]

or a phosphonium salt of the general formula:

$$Ph_3P^+-CH_2-X^3-COOH\cdot Br^- \qquad (VIIb)$$

[wherein $X^3$ represents a methylene group or an ethylene group, and Ph represents the same meaning as hereinbefore defined].

Wittig reaction is well known one, and may be carried out with using an aldehyde compound and a phosphorane compound or phosphonium salt, at a temperature from -78°C to a reflux temperature of a solvent, in an inert organic solvent such as an ether e.g. diethyl ether, tetrahydrofuran, dioxane, 1,2-dimethoxyethane, a hydrocarbon e.g. benzene, toluene, xylene, hexane, a dialkylsulfoxide e.g. dimethylsulfoxide, dialkylformamide e.g. N,N-dimethylformamide, a haloalkane e.g. methylene chloride, chloroform, or an alkanol containing from 1 to 4 carbon atom(s) e.g. methanol, ethanol, or a mixture contained two or more of them.

The Wittig reagents represented by the formulae (VIa), (VIIa), (VIb) and (VIIb) are known per se or may be prepared by known method. [cf. Kagaku no Ryoiki 20, 492 (1966) and Organic Reactions 14, 270 (1965)].

In Wittig reaction, depending upon which reagent is used, trans-olefin, cis-olefin or mixture thereof are produced. That is, when a stable ylide is used, trans-olefin will be produced selectively, and when an unstable ylide is used, a mixture of trans-olefin and cis-olefin will be produced. These mixtures may be separated by known purification means, for example, distillation at atmospheric pressure or reduced pressure, high-speed liquid chromatography or column chromatography using silica gel or recrystallization.

If necessary, carboxylic acid compounds and ester compounds of the general formulae (IIa) and (IIb), obtained by the Wittig reaction, may by converted into esters by esterification and into carboxylic acid compounds by saponification, respectively.

Compounds of the general formula (IV) are known <u>per se</u>, or may be prepared by known methods. For example, 4-(2-carboxyvinyl)-2-methoxyphenol is available as a commercial product.

Compounds of the general formula (V) may be prepared by nitration by methods depicted before, of a compound of general formula:

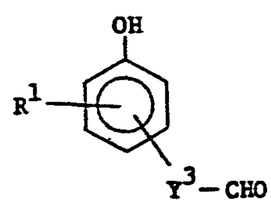

(VIII)

[wherein all of the symbols represent the same meaning as hereinbefore defined].

Compounds of the general formula (IX) may be prepared by nitration by methods depicted before, of a compound of general formula:

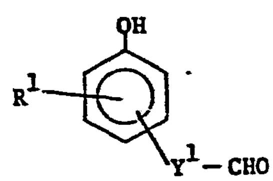

(X)

[wherein all of the symbols represent the same meaning as hereinbefore defined].

Compounds of the general formula (X) may be prepared by Wittig reaction of a compound of the general formula (VIII) and a compound of the general formula (VIb) or (VIIb) to obtain a compound of general formula:

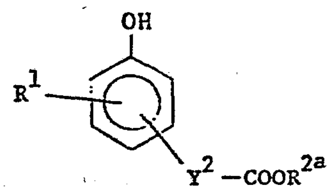

(XI)

[wherein $Y^2$ represents a straight alkenylene group containing from 2 to 4 carbon atoms, $R^1$ and $R^{2a}$ represent the same meaning as hereinbefore defined, and the attached positions of $R^1$ and $Y^2$-$COOR^{2a}$ apply correspondingly as the ones of $R^1$ and Z-$COOR^2$ hereinbefore depicted].
and further by the reduction by the method of reduction B of the straight alkenylene group of the resulting compound of general formula (XI), and to obtain compounds of general formula:

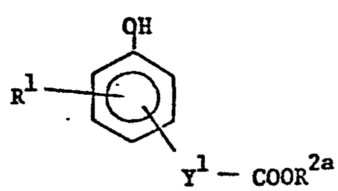

(XII)

[wherein $R^1$, $R^{2a}$ and $Y^1$ represent the same meaning as hereinbefore defined, and the attached positions of $R^1$ and $Y^1$-$COOR^{2a}$ apply correspondingly as the ones of $R^1$ and Z-$COOR^2$ hereinbefore depicted].
and further by selective reduction of the $COOR^{2a}$ group of the compounds of the general formula (XII) into a formyl group.

The selective reduction of the $COOR^{2a}$ group into a formyl group may be carried out, for example, with using diisobutylaluminium hydride

(DIBAL) in hexane, tetrahydrofuran or toluene.

Compounds of the general formula (VIII) are known per se (e.g. 5-chloro-2-hydroxybenzaldehydr is available as a commercial product), or prepared by methods known per se, for example, compounds wherein $R^1$ represents a phenylthio group or an alkylphenylthio group i.e. the compounds represented by general formulae (VIIIa) and (VIIIb) may be prepared by the series of reactions depicted schematically below in Scheme B, wherein A and A' represent a halogen atom such as chlorine, bromine or iodine atom, and symbol Me represents a methyl group.

Scheme B

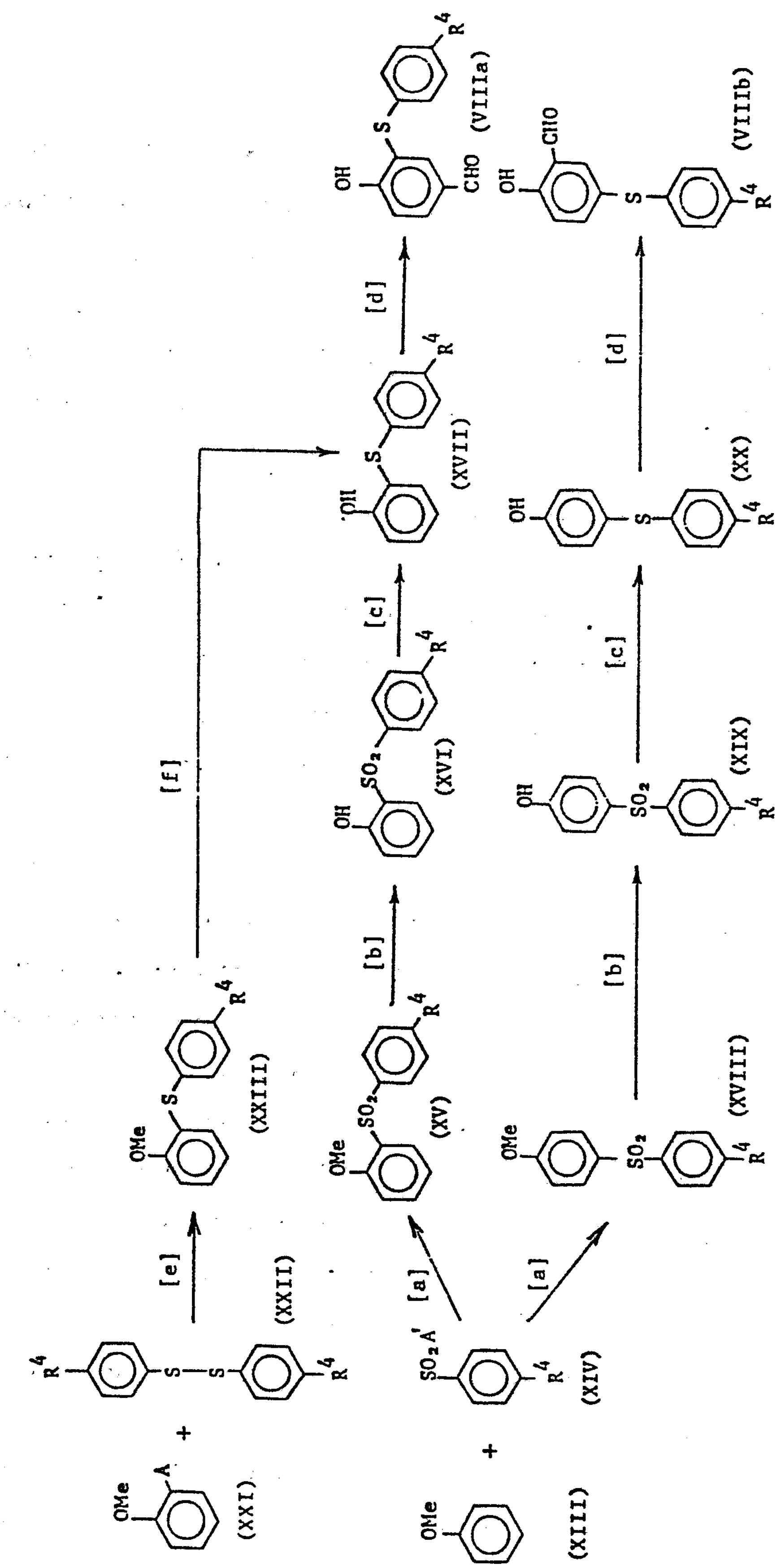


(XXI) (XXII) (XXIII) (XIII) (XIV) (XV) (XVI) (XVII) (VIIIa) (XVIII) (XIX) (XX) (VIIIb)

In Scheme B, each step can be effected using methods known *per se*. For example, step [a] may be carried out by sulfonation like Friedel-Crafts reaction, by reacting with a sulfonating reagent such as sulfonyl halides of the general formula (XIV) in an inert solvent such as chloroform, methylene chloride, carbontetrachloride in the presence of a catalyst such as aluminium chloride at a temperature from -20°C to 50°C.

In step [a], two isomers i.e. the compounds of the general formulae (XV) and (XVIII) may be obtained at the same time, and therefore they must be separated. The separation may be carried out by known purification methods, for example, distillation at atomospheric pressure or reduced pressure, high-speed liquid chromatography, thin layer chromatography or column chromatography using silica-gel or magnesium siliconate, or recrystallization.

Step [b] may be carried out by using hydrobromic acid or a mixture of hydrobromic acid and hydroiodic acid in acetic acid or without solvent, with refluxing. More, according to reaction condition, step [b] may not be necessary; i.e. the compounds of the general formulae (XVI) and (XIX) may be obtained only by the reaction step [a].

Step [c] may be carried out by using a reducing reagent such as diisobutylaluminium hydride (DIBAL) in an inert solvent such as toluene, at a temperature from -20°C to room temperature.

Step [d] may be carried out by formylation, by using chloroform and alkalimetal hydroxide such as sodium hydroxide,in the presence of trialkylamine such as triethylamine or tributylamine, in an inert solvent such as benzene at a temperature from 0°C to 30°C.

Step [e] may be carried out by using a lithioating reagent such as n-butyl lithium in an inert solvent such as tetrahydrofuran at a temperature of -80°C to -40°C.

Step [f] may be carried out by hydrolysis, by using boron tribromide in an inert solvent such as methylene chloride at a temperature of -20°C to 30°C.

In the compounds of the general formula (I), (Ia) and (Ib), the carboxylic acid compounds and the ester compounds can be converted into each other by esterification or saponification, when necessary.

The general synthesis routes above-mentioned are depicted as an example, so any different routes may be thought.

Acid addition salts of 2-aminophenol derivatives of the general formula (I) may be prepared by known methods, for example, by reacting stoichiometric quantities of a compound of the general formula (I) and an appropriate acid e.g. an inorganic acid such as hydrochloric acid, hydrobromic acid, hydroiodic acid, sulfuric acid, phosphoric acid or nitric acid, or an organic acid such as acetic acid, lactic acid, tartaric acid, benzoic acid, citric acid, methanesulfonic acid, ethanesulfonic acid, benzenesulfonic acid, toluenesulfonic acid or isethionic acid, in a suitable solvent.

Preferably, acid addition salts are non-toxic salts. By the term 'non-toxic salts' as used in this specification, is meant salts the anions of which are relatively innocuous to animal organism when used in therapeutic doses so that the benefitical pharmacological properties of the compounds of general formula (I) are not vitiated by side effects ascribable to those anions. Preferably the salts are water-soluble.

Suitable acid addition salts are, for example, inorganic acid salts such as hydrochloride, hydrobromide, hydroiodide, sulfate, phosphate, nitrate and organic acid salts such as citrate, methanesulfonate, ethanesulfonate, benzenesulfonate or toluenesulfonate, isethionate.

Compounds of the general formula (I) and pharmaceutically

acceptable acid addition salts thereof possess an inhibitory effect on 5-lipoxygenase specifically, or on 5-lipoxygenase and cyclooxygenase at the same time, and therefore, useful when it is desired to control the biosynthesis of leukotrienes and various PGs in mammals including humans, especially humans.

For example, in a standard assay system using polymorphonuclear leukocytes from guinea pig (protocols were described hereafter), the concentrations for 50% inhibitions on 5-lipoxygenase and on cyclooxygenase by (a) 2-amino-6-(2-ethoxycarbonylvinyl)phenol hydrochloride were 0.5 μM and 1 μM, respectively, by (b) 2-amino-4-(2-ethoxycarbonylvinyl)phenol hydrochloride were 0.6 μM and 2.2 μM, respectively, by (c) 2-amino-4-[2-[4-biphenylmethoxycarbonyl]-(1E)-vinyl]phenol were 0.8 μM and 10 μM, respectively, by (d) 2-amino-6-[2-ethoxycarbonyl-(1E)-vinyl]-4-phenylthiophenol hydrochloride were 0.2 μM and 0.6 μM, respectively. Therefore, it is comfirmed that the compound (a) inhibits both enzymes at the same time at a very low concentration, and that the compounds (b), (c) and (d) inhibit 5-lipoxygenase slightly specifically at a very low concentration.

The experiments for screening using polymorphonuclear leukocytes were carried out as follows: The reaction mixture (0.1 ml) containing 5 μmole of potassium phosphase at pH 7.4, 0.1 μmole of calcium chloride, polymorphonuclear leukocytes prepared from guinea pigs (4 x $10^7$ cells) and test compound was preincubated for 5 minutes at 37°C. The reaction was started by the addition of [1-$^{14}$C] arachidonic acid (2.2 x $10^5$ dpm, 1.8 μmole), followed by the incubation at 37°C for 5 minutes. The reaction was terminated by the addition of 0.3 ml of a mixture of ether/methanol/1M citric acid (30/4/1). The organic phase (20 μl) was applied to silica gel plate. Thin layer chromatography was performed to separate each product with solvent system of ether/petroleum ether/acetic acid (85/15/0.1).

Activities of 5-lipoxygenase and cyclooxygenase were calculated from the measurements of the productions of 5-HETE, and 12L-hydroxy-5, 8, 10-heptatrienoic acid (i.e. HHT) or thromboxane $B_2$ (i.e. $TXB_2$) respectively. [it has been known that HHT and $TXB_2$ were the last products of the system concerning cyclooxygenase in the arachidonate cascade.] The concentrations for 50% inhibitions on 5-lipoxygenase and on cyclooxygenase were evaluated by using the concentrations of compounds of the present invention required 50% inhibitions on the productions of 5-HETE, and HHT or $TXB_2$.

Further, in another assay system, the inhibitory activity of compounds of the present invention on 5-lipoxygenase only was also evaluated by using an enzyme prepared from polymorphonuclear leukocytes of guinea pig. Guinea pig was treated by casein and harvested polymorphonuclear leukocytes from cavity were sonicated, followed by the centrifugation at 105,000 x g. The surpernate was used as an enzyme throughout this work. The supernate (100 - 500 μg of protein) was incubated for 5 minutes at 30°C with [$^{14}$C] arachidonic acid and 1 mM calcium chloride in the presence or in the absence of test compound. The reaction was terminated by the addition of ether. Ether extract was separated by thin layer chromatography using silica gel plates to observe production of 5-HETE, 5,12-diHETE and LTB.

In this assay, the concentrations of 2-amino-4-chloro-6-[2-ethoxycarbonyl-(1E)-vinyl]phenol hydrochloride, 2-amino-4-chloro-6-[4-ethoxycarbonyl-(1E,3E)-butadienyl]phenol hydrochloride, 2-amino-6-methoxy-4-[2-methoxycarbonyl-(1E)-vinyl]phenol hydrochloride, 2-amino-4-[2-[4-biphenylmethoxycarbonyl]-(1E)-vinyl]phenol and 2-amino-6-[2-ethoxycarbonyl-(1E)-vinyl]-4-phenylthiophenol hydrochloride required to produce a 50% inhibition on the productions of 5-HETE, 5,12-diHETE and LTB (concentration for a 50% inhibition of 5-lipoxygenase, i.e. $IC_{50}$) were 0.6 μM, 0.6 μM, 2.7 μM, 0.3 μM and 0.22 μM, respectively.

On the other hand, all extent of the compounds of the present invention were confirmed that their acute toxicities were more than 30 mg/Kg by intravenous injection. Therefore, the compounds of the present invention may be considered to be sufficiently safe and suitable for medical use.

For example, in a test of acute toxicity using mice and administering by intravenous injection to their tail vein, 2-amino-6-methoxy-4-[2-methoxycarbonyl-(1E)-vinyl]phenol hydrochloride, 2-amino-4-chloro-6-[2-ethoxycarbonyl-(1E)-vinyl]phenol hydrochloride and 2-amino 4-chloro-6-[4-ethoxycarbonyl-(1E,3E)-butadienyl]phenol hydrochloride possessed no dead case in 21 cases at doses of 30 mg/Kg.

To control the biosynthesis of leukotrienes and prostaglandins are useful for the prevention and the treatment of allergic tracheal and bronchial diseases or allergic lung diseases, allergic shocks or various allergic inflammations, in addition to various inflammations induced by prostaglandins in mammals including human , especially human. For such purposes, the compounds of this invention are usually administered systemically, for example, orally, rectally or parenterally. Doses are determined depending upon age, symptoms, the desired therapeutic effects, the routes of administration, the duration of the treatment and the like, and are generally and preferably about between 0.1 mg and 500 mg for oral administration; and between 1 μg and 1 mg for intravenous injection or between 0.1 μg and 0.1 mg/hour for intravenous infusion, specially when required emergency treatment.

Solid compositions for oral administration include compressed tablets, pills, dispersible powders and granules. In such solid compositions,

one or more of the active compounds is or are, admixed with at least one inert diluent such as calcium carbonate, potato starch, dextrin, alginic acid, lactose, mannitol, glucose or cacao butter. The compositions may also comprise, as is normal practice, additional substances other than inert diluents e.g. lubricating agents such as magnesium stearate and disintegrating agents such as cellulose calcium gluconate. The tablets or pills may, if desired, be coated and made into sugar-coated, gelatin-coated, enteric-coated or film-coated tablets and pills, or tablets or pills may be coated with two or more layers.

Liquid compositions for oral administration include pharmaceutically acceptable emulsions, solutions, suspensions, syrups and elixirs, containing inert diluents such as water or liquid paraffin, commonly used in the art. Besides inert diluents, such compositions may also comprise adjuvants such as wetting and suspending agents, and sweetening, flavouring, perfuming and preserving agents.

The compositions according to this invention for oral administration, also include capsules of absorbable materials such as gelatin, containing one or more of the active ingredients with or without the addition of diluents or excipients.

Solid compositions for intrarectal administration include suppositories containing one or more active ingredients, formulated in known manner.

Preparations according to this invention for parenteral administration include sterile aqueous or non-aqueous solutions, suspensions and emulsions. Examples of non-aqueous solvents or suspending media are, for example, propylene glycol, polyethylene glycol, ethanol, vegetable oils, such as olive oil, and injectable organic esters such as ethyl oleate,

sorbitan esters. These compositions may also include adjuvants such as preserving, wetting, emulsifying and dispersing agents. They may be sterilized, for example, by filtration through a bacteria-retaining filter, by incorporation of sterilizing agents in the composition or by irradiation. They may also be manufactured in the form of sterile solid compositions, and which can be used to dissolve in sterile water or some other media, immediately before use.

The proportions of active ingredient in the compositions of the present invention may be varied, and it is required to constitute a proportion such that a suitable dosage for the desired therapeutic effect shall be obtained. Several unit dosage forms may of course be administered at the same time.

In general, the preparations for injection contain normally at least 0.025% of active ingredients by weight, and ones for oral administration contain normally at least 0.1% of active ingredients by weight.

Preferable compounds included this invention, for example, the following compounds and pharmaceutically acceptable non-toxic acid addition salts thereof may be listed; included in the general formula (Ia):

2-amino-4-methoxycarbonylmethylphenol,

2-amino-4-ethoxycarbonylmethylphenol,

2-amino-6-chloro-4-methoxycarbonylmethylphenol,

2-amino-6-chloro-4-ethoxycarbonylmethylphenol,

2-amino-6-methoxy-4-methoxycarbonylmethylphenol,

2-amino-4-ethoxycarbonylmethyl-6-methoxyphenol,

2-amino-4-methoxycarbonylethylphenol,

2-amino-4-ethoxycarbonylethylphenol,

2-amino-6-chloro-4-methoxycarbonylethylphenol,

2-amino-6-chloro-4-ethoxycarbonylethylphenol,

2-amino-6-methoxy-4-methoxycarbonylethylphenol,

2-amino-4-ethoxycarbonylethyl-6-methoxyphenol,

2-amino-4-(2-methoxycarbonylvinyl)phenol,

2-amino-4-(2-ethoxycarbonylvinyl)phenol,

2-amino-6-chloro-4-(2-methoxycarbonylvinyl)phenol,

2-amino-6-chloro-4-(2-ethoxycarbonylvinyl)phenol,

2-amino-6-methoxy-4-(2-methoxycarbonylvinyl)phenol,

2-amino-4-(2-ethoxycarbonylvinyl)-6-methoxyphenol,

2-amino-4-(2-methoxycarbonyl-1-propenyl)phenol,

2-amino-4-(2-ethoxycarbonyl-1-propenyl)phenol,

2-amino-6-chloro-4-(2-methoxycarbonyl-1-propenyl)phenol,

2-amino-6-chloro-4-(2-ethoxycarbonyl-1-propenyl)phenol,

2-amino-6-methoxy-4-(2-methoxycarbonyl-1-propenyl)phenol,

2-amino-4-(2-ethoxycarbonyl-1-propenyl)-6-methoxyphenol,

2-amino-4-(4-methoxycarbonylbutadienyl)phenol,

2-amino-4-(4-ethoxycarbonylbutadienyl)phenol,

2-amino-6-chloro-4-(4-methoxycarbonylbutadienyl)phenol,

2-amino-6-chloro-4-(4-ethoxycarbonylbutadienyl)phenol,

2-amino-6-methoxy-4-(4-methoxycarbonylbutadienyl)phenol,

2-amino-4-(4-ethoxycarbonylbutadienyl)-6-methoxyphenol,

2-amino-6-(2-carboxyvinyl)phenol,

2-amino-4-t-butyl-6-(2-ethoxycarbonylethyl)phenol,

2-amino-4-t-butyl-6-(2-methoxycarbonylethyl)phenol,

2-amino-4-t-butyl-6-(2-ethoxycarbonylpropyl)phenol,

2-amino-4-t-butyl-6-(2-ethoxycarbonylvinyl)phenol,

2-amino-4-t-butyl-6-(2-methoxycarbonylvinyl)phenol,

2-amino-4-t-butyl-6-(2-ethoxycarbonylpropenyl)phenol,

2-amino-6-(2-ethoxycarbonylvinyl)-4-phenylthiophenol,

2-amino-6-(2-methoxycarbonylvinyl)-4-phenylthiophenol,

2-amino-6-(2-carboxyvinyl)-4-phenylthiophenol,

2-amino-6-(2-ethoxycarbonylvinyl)-4-(4-pentylphenylthio)phenol,

2-amino-6-(2-methoxycarbonylvinyl)-4-(4-pentylphenylthio)phenol,

2-amino-6-(2-carboxyvinyl)-4-(4-pentylphenylthio)phenol,

2-amino-6-(2-ethoxycarbonylpropenyl)-4-phenylthiophenol,

2-amino-6-(2-methoxycarbonylpropenyl)-4-phenylthiophenol,

2-amino-6-(2-carboxypropenyl)-4-phenylthiophenol,

2-amino-6-(2-ethoxycarbonylpropenyl)-4-(4-pentylphenylthio)phenol,

2-amino-6-(2-methoxycarbonylpropenyl)-4-(4-pentylphenylthio)phenol and

2-amino-6-(2-carboxypropenyl)-4-pentylphenylthiophenol;

and included in the general formula (Ib):-

2-amino-6-methoxycarbonylmethylphenol,

2-amino-6-ethoxycarbonylmethylphenol,

2-amino-4-chloro-6-methoxycarbonylmethylphenol,

2-amino-4-chloro-6-ethoxycarbonylmethylphenol,

2-amino-4-methoxy-6-methoxycarbonylmethylphenol,

2-amino-6-ethoxycarbonylmethyl-4-methoxyphenol,

2-amino-6-methoxycarbonylethylphenol,

2-amino-6-ethoxycarbonylethylphenol,

2-amino-4-chloro-6-methoxycarbonylethylphenol,

2-amino-4-chloro-6-ethoxycarbonylethylphenol,

2-amino-4-methoxy-6-methoxycarbonylethylphenol,

2-amino-6-ethoxycarbonylethyl-4-methoxyphenol,

2-amino-6-(2-methoxycarbonylvinyl)phenol,

2-amino-6-(2-ethoxycarbonylvinyl)phenol,

2-amino-4-chloro-6-(2-methoxycarbonylvinyl)phenol,

2-amino-4-chloro-6-(2-ethoxycarbonylvinyl)phenol,

2-amino-4-methoxy-6-(2-methoxycarbonylvinyl)phenol,

2-amino-6-(2-ethoxycarbonylvinyl)-4-methoxyphenol,

2-amino-6-(2-methoxycarbonyl-1-propenyl)phenol,

2-amino-6-(2-ethoxycarbonyl-1-propenyl)phenol,

2-amino-4-chloro-6-(2-methoxycarbonyl-1-propenyl)phenol,

2-amino-4-chloro-6-(2-ethoxycarbonyl-1-propenyl)phenol,

2-amino-4-methoxy-6-(2-methoxycarbonyl-1-propenyl)phenol,

2-amino-6-(2-ethoxycarbonyl-1-propenyl)-4-methoxyphenol,

2-amino-6-(4-methoxycarbonylbutadienyl)phenol,

2-amino-6-(4-ethoxycarbonylbutadienyl)phenol,

2-amino-4-chloro-6-(4-methoxycarbonylbutadienyl)phenol,

2-amino-4-chloro-6-(4-ethoxycarbonylbutadienyl)phenol,

2-amino-4-methoxy-6-(4-methoxycarbonylbutadienyl)phenol,

2-amino-6-(4-ethoxycarbonylbutadienyl)-4-methoxyphenol,

2-amino-4-[2-(4-biphenylmethoxycarbonyl)vinyl]phenol,

2-amino-4-[2-(4-biphenylmethoxycarbonyl)vinyl]-6-chlorophenol and

2-amino-4-[2-(4-biphenylmethoxycarbonyl)vinyl]-6-methoxyphenol.

The following Reference Examples and Examples illustrate, but not limit, the preparation of compounds of the present invention. In the Reference Examples and Examples, 'mp', 'TLC', 'IR', 'NMR' and 'Mass' represent 'melting point', 'Thin layer chromatography', 'Infrared absorption spectrum', 'Nuclear magnetic resonance' and 'Mass spectrum', respectively.

Where solvent ratios are specified in chromatographic separations, the ratios are by volume: the solvents in parentheses in thin layer chromatography show the developing solvent used. Except when specified otherwise, infrared spectra are recorded by KBr tablet method and nuclear magnetic resonance spectra are recorded in deuterochloroform ($CDCl_3$) solutions.

Reference Example 1

2-[2-Carboxy-(1E)-vinyl]-6-nitrophenol

To a solution of 2.0 g of 2-[2-carboxy-(1E)-vinyl]phenol (commercial product) in 20 ml of acetic acid, 0.77 g of concentrated nitric acid was added dropwise at room temperature. In this solution, crystals were deposited after stirring for one hour. After addition of ice-water, the reaction solution was filtered, and gathered crystals were washed with water, and dried in vacuo by vacuum pump to give 2.1 g of crystals of the title compound (p-nitro isomer was partly contained) having the following physical characteristic:

NMR(deuterochloroform + acetone-$d_6$ + dimethylsulfoxide-$d_6$):

$\delta$ = 8.3 - 7.6(6H, m), 7.0(1H, d), 6.99(1H, t), 6.57(1H, d), 6.53(1H, d).

The next compounds having the following physical characteristics were given by the same procedure as described in Reference Example 1:

(a) 5-Chloro-2-hydroxy-3-nitrobenzaldehyde

Starting material: 1.6 g of 5-chlorosalicylaldehyde (commercial product);

Yield: 1.9 g (yellow crystals);

mp: 112° - 113°C;

TLC(methylene chloride): Rf = 0.25;

IR: ν = 3430, 1650, 1523, 1440, 1365, 1345, 1300, 1263, 1223, 955, 915, 815, 772, 738, 710 $cm^{-1}$;

NMR(deuterochloroform + acetone-$d_6$):

δ = 10.23(1H, s), 8.21(1H, d), 7.98(1H, d).

(b) 4-Carboxymethyl-2-nitrophenol

Starting material: 1.52 g of p-hydroxyphenyl-acetic acid (commercial product);

Yield: 1.37 g;

TLC (methylene chloride : methanol = 10:1): Rf = 0.2;

IR: ν = 3450, 3280, 3090, 3050, 2970, 2760, 2670, 1700, 1630, 1580, 1530, 1490, 1430, 1410, 1330, 1305, 1260, 1215, 1175, 1130, 1085, 920, 845, 825, 765, 645 $cm^{-1}$;

NMR(deuterochloroform + methanol-$d_4$):

δ = 7.92 (1H, d), 7.45(1H, dd), 7.03(1H, d), 3.57(2H, s);

Mass: m/e = 197($M^+$), 181, 152, 135, 106.

(c) 4-[2-Carboxy-(1E)-vinyl]-2-nitrophenol

Starting material: 1.64 g of p-hydroxy-trans-cynnamic acid (commercial product);

Yield: 630 mg;

NMR: δ = 8.07(1H, d), 7.67(1H, dd), 7.46(1H, d), 7.08(1H, d), 6.32(1H, d);

Mass: m/e = 209($M^+$), 192, 162, 134, 118.

(d) 4-[2-carboxy-(1E)-vinyl]-2-methoxy-6-nitrophenol

Starting material: 1.46 g of 4-[2-carboxy-(1E)-vinyl]-2-methoxyphenol (commercial product);

Yield: 450 mg;

NMR(acetone-d + methanol-$d_4$):

$\delta$ = 7.70(1H, d), 7.50(1H, d), 7.45(1H, d), 6.43(1H, d), 3.95(3H, s);

Mass: m/e = 239($M^+$), 194, 138, 123, 105.

Reference Example 2

2-[2-Ethoxycarbonyl-(1E)-vinyl]-6-nitrophenol

The solution of 2.1 g of 2-[2-carboxy-(1E)-vinyl]-6-nitrophenol (prepared in Reference Example 1) dissolved in 30 ml of ethanol, 1 ml of concentrated hydrochloric acid was added, and the solution was refluxed for 16 hours. The solution was concentrated under reduced pressure and further dried in vacuo by vacuum pump. The residue was purified by column chromatography on silica gel using a mixed solvent of chloroform and ethyl acetate (5:1) as an eluent to give 1.1 g of the title compound having the following physical characteristics:

TLC(chloroform: ethyl acetate = 3:1): Ref = 0.65;

IR : $\nu$ = 3430, 3200, 3100, 3000, 2900, 1715, 1640, 1600, 1585, 1535, 1460, 1440, 1390, 1365, 1325, 1270, 1190, 1160, 1145, 1095, 1025, 990, 980, 925, 870, 840, 810, 740, 685 $cm^{-1}$;

NMR: $\delta$ = 11.20(1H, s), 8.03(1H, dd), 7.80(1H, d), 7.70(1H, dd), 6.92(1H, t), 4.23(2H, q), 1.33(3H, t);

Mass: m/e = 237($M^+$), 191, 174, 164, 148, 118, 89.

Reference Example 3

4-Chloro-2-[2-ethoxycarbonyl-(1E)-vinyl]-6-nitrophenol

At room temperature, 403 mg of 5-chloro-2-hydroxy-3-nitrobenz aldehyde (prepared in Reference Example 1(a).), 767 mg of ethoxycarbonyl-methylidene triphenylphosphorane and 4 ml of toluene were mixed.

The solution was allowed to stand for 25 minutes and purified by column chromatography on silica gel using a mixed solvent of n-hexane and methylene chloride (1 : 1) as an eluent to give 476 mg of the title compound having the following physical characteristics:

mp: 110°C - 112°C;

TLC(n-hexane: methylene chloride = 1:1): Rf = 0.24;

IR : ν = 3420, 3240, 1705, 1605, 1521, 1446, 1416, 1265, 1240, 1150, 1040, 983, 900, 732 $cm^{-1}$;

NMR: δ = 11.0(1H, s), 8.03(1H, d), 7.80(1H, d), 7.68(1H, d), 6.53(1H, d), 4.25(2H, q), 1.33(3H, t).

Reference Example 4

4-Chloro-2-[2-formyl-(1E)-vinyl]-6-nitrophenol

At room temperature, 443 mg of 5-chloro-2-hydroxy-3-nitrobenzaldehyde (prepared in Reference Example 1(a).), 608 mg of formylmethylene triphonylphosphorane and 10 ml of methylene chloride were mixed. The solution was stirred for 1.5 hours. After that, the solution was purified by column chromatography on silica gel using methylene chloride as an eluent to give 455 mg of the title compound (unreacted starting material was partly contained.) having the following physical characteristic:

TLC(methylene chloride): Rf=0.33.

Reference Example 5

4-Chloro-2-[4-ethoxycarbonyl-(1E, 3E)-butadienyl]-6-nitrophenol

At room temperature, 455 mg of 4-chloro-2-[2-formyl-(1E)-vinyl]-6-nitrophenol (prepared in Reference Example 4), 766 mg of ethoxycarbonylmethylidene triphenylphosphorane and 7 ml of methylene chloride were mixed. The solution was stirred at 50°C for 2 hours. The solution was cooled to

room temperature and concentrated under reduced pressure. The residue was purified by column chromatography on silica gel using a mixed solvent of n-hexane and methylene chloride (1:1) as an eluent to give 511 mg of the title compound having the following physical characteristics:

TLC(methylene chloride : n-hexane = 1:1): Rf=0.22;

IR : ν = 3400, 1700, 1620, 1522, 1445, 1403, 1330, 1313, 1303, 1240, 1165, 1137, 1027, 1000, 892, 862, 761, 722, 713 $cm^{-1}$;

NMR: δ = 10.91(1H, s), 7.91(1H, d), 7.63(1H, d), 7.60-6.10 (3H, m), 5.98(1H, d), 4.19(2H, q), 1.31(3H, t).

Reference Example 6

4-Chloro-2-[3-methoxycarbonyl-(1E)-propenyl]-6-nitrophenol

To a solution of 5.0 g of (2-carboxyethyl)-triphenylphosphonium bromide (commercial product) in 20 ml of dimethyl sulfoxide was added 25 ml of 1M solution of sodium methylsulphinyl methylide in dimethyl sulfoxide which was prepared independently, at room temperature. Five minutes' after, a solution of 0.95 g of 5-chloro-2-hydroxy-3-nitrobenzaldehyde (prepared in Reference Example 1(a).) dissolved in 9 ml of dimethyl sulfoxide was added to the solution. The solution was stirred for 1 hour at room temperature and added 150 ml of water and adjusted to pH2 with 1N hydrochloric acid. The reaction solution was extracted with ethyl ether, the extract was washed with water and a saturated aqueous solution of sodium chloride, dried over anhydrous sodium sulfate and concentrated under reduced pressure. The residue was dissolved in 20 ml of methanol, and dropped a solution of diazomethane in ethyl ether at 0°C. Dropping was stopped after confirmation of disappearance of

starting material on TLC. The reaction solution was concentrated under reduced pressure, and purified by column chromatography on silica gel using a mixed solvent of n-hexane and methylene chloride (3:2) as an eluent to give 722 mg of crude title compound. Obtained compound was purified by recrystallization from a mixture of n-hexane and methylene chloride (10:1) to give 290 mg of the title compound having the following physical characteristics:

mp : 122° - 124°C;

TLC(n-hexane: ethyl ether =3:2):Rf = 0.41;

IR : ν = 3200, 1730, 1643, 1600, 1570, 1520, 965, 900, 723, 690 $cm^{-1}$;

NMR: δ = 10.94(1H, s), 8.03(1H, d), 7.73(1H, d), 6.84(1H, d), 6.47(1H, dt), 3.76(3H, s), 3.35(2H, d).

Reference Example 7

4-Methoxycarbonylmethyl-2-nitrophenol

To 420 mg of 4-Carboxymethyl-2-nitrophenol(prepared in Reference Example 1(b).), 20 ml of acetonedimethylacetal and 1 ml of concentrated hydrochloric acid was added and the mixture was stirred for 16 hours. The solution was concentrated under reduced pressure and dried in vacuo by vacuum pump. The residue was purified by column chromatography on silica gel using methylene chloride as an eluent to give 450 mg of the title compound having the following physical characteristics:

TLC(benzene: ethyl acetate = 10:1): Rf = 0.52;

IR : ν = 3400, 3090, 2970, 2850, 1730, 1630, 1575, 1535, 1480, 1435, 1425, 1350, 1320, 1275, 1240, 1195, 1080, 990, 915, 840, 820, 800, 765, 730 $cm^{-1}$;

NMR : δ = 10.35(1H, s), 7.91(1H, d), 7.48(1H, dd), 7.03(1H, d), 3.68(3H, s), 3.60(2H, s);

Mass: m/e = 211($M^+$), 152, 135, 106, 105.

The next ester compounds having the following physical characteristics were given from the corresponding carboxylic acids by the same procedure as described in Reference Example 7:

(a) 2-Methoxy-4-[2-methoxycarbonyl-(1E)-vinyl]-6-nitrophenol

Starting material : 450 mg of 4-[2-carboxy-(1E)-vinyl]-2-methoxy-6-nitrophenol (prepared in Reference Example 1(d).);

Yield: 307 mg;

TLC(benzene: ethyl acetate = 10:1) : Rf = 0.36;

IR : ν = 3450, 3220, 3080, 2950, 1710, 1635, 1610, 1540, 1440, 1385, 1320, 1300, 1270, 1245, 1200, 1170, 1140, 1065, 1000, 925, 860, 770, 740 $cm^{-1}$;

NMR : δ = 10.77(1H, s), 7.77(1H, d), 7.50(1H, d), 7.17(1H, d), 6.33(1H, d), 3.93(3H, s), 3.78(3H, s);

Mass : m/e = 253($M^+$), 222, 213, 204, 192, 133.

<u>Reference Example 8</u>

4-[2-Ethoxycarbonyl-(1E)-vinyl]-2-nitrophenol

To a solution of 630 mg of 4-[2-Carboxy-(1E)-vinyl]-2-nitrophenol dissolved in 20 ml of ethanol, 5 ml of concentrated hydrochloric acid was added, and the solution was refluxed for 18 hours. After cooling and addition of water, the solution was stirred. Deposited crystals were collected by filtration and washed with water, and dried in vacuo by vacuum pump to obtain 445 mg of the title compound having the following

physical characteristics:

TLC (methylene chloride : methanol = 10:1): Rf = 0.82;

IR : ν = 3450, 3070, 2990, 2920, 1720, 1640, 1625, 1530, 1425, 1315, 1295, 1250, 1180, 1140, 1030, 990, 830 $cm^{-1}$;

NMR : δ = 10.6(1H, s), 8.13(1H, d), 7.67(1H, dd), 7.48(1H, d), 7.13(1H, d), 6.33(1H, d), 4.23(2H, q), 1.31(3H, t);

Mass: m/e = 237($M^+$), 209, 192, 165, 146, 118, 89.

The next compound having the following physical characteristics was given by the same procedure as described in Reference Example 8:

(a) 4-[2-Ethoxycarbonyl-(1E)-vinyl]-2-methoxy-6-nitrophenol.

Starting material: 1.16 g of 4-[2-carboxy-(1E)-vinyl]-2-methoxy-6-nitrophenol (prepared in Reference Example 1(d).);

Yeild: 155 mg (by-product was partly contained);

NMR : δ = 7.67(1H, d), 7.43(2H, d), 7.13(1H, d), 6.90(3H, m), 6.30(1H, d), 6.20(1H, d), 4.20(2H, q), 4.18(2H, q), 3.90(3H, s), 3.83(3H, s), 1.32(6H, t).

Referemce Example 9

5-t-Butyl-2-hydroxybenzaldehyde

In an atomosphere of nitrogen, to a solution of 30 g of 4-t-butylphenol dissolved in 300 ml of trifluoroacetic acid, 28 g of hexamethylenetetramine was added slowly. The solution was refluced for 18 hours. After cooling, the solution was concentrated. The solution was poured into 1.2 liter of ice-water. The solution was neutralized with sodium carbonate, and extracted with ethyl ether. The extract was dried and

concentrated. The residue was purified by column chromatography on silica gel using a mixture of chloroform and cyclohexane (3:7) as an eluent to give 18.77 g of the title compound having the following physical characteristics:

TLC(chloroform:cyclohexane = 1:1): Rf = 0.38;

IR(liquid film): ν = 2950, 1650, 1480, 1285 1260, 1230, 1180 $cm^{-1}$;

NMR: δ = 10.89(1H, s), 9.92(1H, s), 7.62(1H, dd), 7.56(1H, s), 6.96(1H, d), 1.34(9H, s);

Mass: m/e = 178($M^+$), 163, 105.

Reference Example 10

5-t-Butyl-2-hydroxy-3-nitrobenzaldehyde

In an atomosphere of nitrogen, to a solution of 5 g of 5-t-butyl-2-hydroxybenzaldehyde (prepared in Reference Example 9) dissolved in 30 ml of acetic acid, 2.14 ml of a 60% nitric acid was added over a period of 30 minutes at 10°C. The solution was stirred for 30 minutes. To the solution, 10 ml of ice-water was added. The depositing crystals were gathered by filtration, washed with water and dried to give 4.29 g of the title compound having the following physical characteristics:

mp: 90°C;

TLC(chloroform): Rf = 0.52;

IR: ν = 3250, 1680, 1620, 1580, 1525, 1410, 1295, 1260, 1145 $cm^{-1}$;

NMR: δ = 11.26(1H, s), 10.45(1H, s), 8.38(1H, d), 8.18(1H, d), 1.38(9H, s);

Mass: m/e = 223($M^+$), 208, 205.

Reference Example 11

4-t-Butyl-2-[2-ethoxycarbonyl-(1E)-vinyl]-6-nitrophenol

A solution of 1 g of 5-t-butyl-2-hydroxy-3-nitrobenzaldehyde (prepared in Reference Example 10) dissolved in 5 ml of chloroform was cooled with ice bath. To the solution, a solution of 1.73 g of ethoxycarbonylvinylidene triphenylphosphorane dissolved in 5 ml of chloroform was added, the solution was stirred for 30 minutes. The reaction mixture was concentrated, and obtained residue was purified by column chromatography on silica gel using a mixture of methylene chloride and n-hexane (2:1) as an eluent to give 1.07 g of the title compound having the following physical characteristics:

NMR: δ = 11.16(1H, s), 8.17(1H, d), 7.99(1H, d), 7.87(1H,d), 6.71(1H, d), 4.31(2H, q), 1.37(3H, t);

Mass: m/e = 293, 278, 248, 247, 232, 204, 186.

Reference Example 12

4-[2-[4-Biphenylmethoxycarbonyl]-(1E)-vinyl]-2-nitrophenol

In an atomosphere of nitrogen, to a solution of 1.12 g of 4-[2-carboxy-(1E)-vinyl]-2-nitrophenol (prepared in Reference Example 1(c)) and 975 mg of 4-biphenylmethanol dissolved in 60 ml of pyridine, 1.01 g of p-toluenesolfonic chloride was added at room temperature. The solution was refluxed for 18 hours with molecular sieves. The solution was cooled, diluted with ethyl ether. The solution was washed with water, dried and concentrated. The residue was purified by column chromatography on silica gel using methylene chloride as an eluent to give 121 mg of the title compound having the following physical characteristics:

mp: 115 - 120°C;

TLC(methylene chloride): Rf = 0.45;

IR: ν = 1705, 1640, 1620, 1530, 1480, 1280, 1160 $cm^{-1}$;

NMR: δ = 10.74(1H, s), 8.27(1H, d), 7.3 - 7.9(12H, m),
7.2(1H, d), 6.48(1H, d), 5.31(2H, s);

Mass: m/e = 375($M^+$), 357, 167.

Reference Example 13

4-(4-Pentylbenzenesulfonyl)anisole

To a suspension of 7.7 g of aluminium chloride in 50 ml of dichloroethane, 10 g of 4-methoxybenzenesulfonyl chloride was added, and the mixture was stirred at room temperature for 30 minutes. To the solution, 11 g of pentylbenzene was added and the solution was stirred at room temperature for 1 hour. The reaction solution was poured into ice-water. The mixture was extracted with methylene chloride. The extract was washed with a 1N aqueous solution of sodium hydroxide, water, a saturated aqueous solution of sodium chloride, successively, dried over anhydrous magnesium sulfate, and concentrated. The residue was purified by column chromatography on silica gel using methylene chloride as an eluent to give 15 g of the title compound having the following physical chracteristics:

IR: ν = 3040, 2950, 2860, 1595, 1495, 1300, 1250, 1150, 1105,
1015, 835, 800 $cm^{-1}$;

NMR: δ = 7.72(2H, d), 7.68(2H,d), 7.20(2H, d), 6.88(2H, d),
3.73(3H, s), 2.60(2H, t), 1.35(6H, m), 0.83(3H, t);

Mass: m/e = 318($M^+$), 262, 155, 123, 91.

The next compound having the following physical characterisrics was given by the same procedure described in Reference Example 13:

(a) 4-Benzenesulfonylanisole

Starting material: 10 g of 4-methoxybenzenesulfonyl chloride and 7.6 g of benzene;

Yield: 11.3 g;

IR: $\nu$ = 3050, 2945, 2840, 1590, 1495, 1440, 1315, 1295, 1260, 1150, 1105, 1070, 1020, 835, 805, 755, 730, 705, 685, 655 $cm^{-1}$;

NMR: $\delta$ = 7.9 - 7.6(4H, m), 7.4 - 7.3(3H, m), 6.88(2H, d), 3.77(3H, s);

Mass: m/e = 248($M^+$), 155, 123.

Reference Example 14

4-(4-Pentylphenylthio)phenol

To a solution of 15 g of 4-(4-pentylbenzenesulfonyl)anisole (prepared in Reference Example 13) in 30 ml of toluene, 107 ml of a 1.76N diisobutylaluminium hydride in toluene was added with cooling with ice bath. After addition, the solution was refluxed for 22 hours. After cooling, an aqueous saturated solution of sodium potassium tartrate was added to the solution. To the mixture, ethyl acetate was added. To the mixture, anhydrous magnesium sulfate was added, and the mixture was stirred well. The mixture was filtered, and the washings and filtrate was concentrated to give 10 g of oily residue. The residue was purified by column chromatography on silica gel using a mixture of ethyl acetate and hexane (1:20) as an eluent to give 7.45 g of the title compound having the following physical characteristics:

IR(liquid film): $\nu$ = 3350, 3025, 2940, 2855, 1595, 1585,

1490, 1230(broad), 830 $cm^{-1}$;

NMR: $\delta$ = 7.18(2H, d), 7.00(4H, s), 6.72(2H, d), 2.50(2H, t), 1.3(6H, m), 0.87(3H, t);

Mass: m/e = 272($M^+$), 215, 183, 125, 91.

The next compound having the following physical characteristics was given by the same procedure described in Reference Example 14:

(a) 4-Phenylthiophenol

Starting material: 11 g of 4-benzenesulfonylanisole (prepared in Reference Example 13(a));

Yield: 4 g;

IR(liquid film): $\nu$ = 3400, 3050, 1600, 1580, 1490, 1475, 1435, 1170, 830, 740, 690 $cm^{-1}$;

NMR: $\delta$ = 7.3 -7.0(7H, m), 6.62(2H, d), 5.80(1H, broad-s);

Mass: m/e = 202($M^+$), 183, 173, 141, 125, 115.

Reference Example 15

2-Formyl-4-(4-pentylphenylthio)phenol

To a solution of 4-(4-pentylphenylthio)phenol (prepared in Reference Example 14) dissolved in 50 ml of benzene, 26.3 g of chloroform and 40 mg of tri-n-butylamine was added. To the solution, 17.7g of a 50% aqueous solution of sodium hydroxide was added over a period of 30 minutes at 60°C. The solution was stirred for 30 minutes at 60°C. After cooling, the solution was adjusted to pH 3 by an addition of diluted sulfuric acid and stirred for 10 minutes. The solution was extracted with ethyl acetate. The extract was washed with water and a saturated aqueous solution of sodium chloride and concentrated. The obtained residue was purified by column chromatography on silica gel

using a mixture of methylene chloride and n-hexane(1:5) as an eluent to give 960 mg of the title compound having the following physical characteristics:

NMR: δ = 10.90(1H, s), 9.68(1H, s), 7.47(2H, m), 7.06(4H, s), 6.87(1H, d), 2.55(2H, t), 1.3(6H, m), 0.87(3H, t);

Mass: m/e = 300($M^+$), 243, 121, 115, 91.

The next compound having the following physical characteristics was given by the same procedure described in Reference Example 15:

(a) 2-Formyl-4-phenylthiophenol

Starting material: 7.9 g of 4-phenylthiophenol (prepared in Reference Example 14(a));

Yield: 2 g;

TLC(benzene:ethyl acetate = 50:1): Rf = 0.73;

NMR: δ = 10.85(1H, s), 9.55(1H, s), 7.46(1H, d), 7.38(1H, dd), 7.08(5H, s), 6.83(1H, d);

Mass: m/e = 230 ($M^+$), 201, 184, 171, 141, 110.

Reference Example 16

2-Formyl-6-nitro-4-(4-pentylphenylthio)phenol

A solution of 1.04 g of 2-formyl-4-(4-pentylphenylthio)phenol (prepared in Reference Example 15) dissolved in 5.5 ml of acetic acid and 5.5 ml of methylene chloride was cooled with ice. To the solution, 405 mg of concentrated nitric acid was added slowly at 0°C, and the solution was stirred for 30 minutes at the same temperature. The solution was extracted with methylene chloride after an addition of ice-water. The extract was washed with an aqueous solution of sodium bicarbonate, water and a saturated aqueous solution of sodium chloride,

successively, dried over anhydrous sodium sulfate, and concentrated. The obtained residue was purified by column chromatography on silica gel using a mixture of n-hexane and ethyl acetate(10:1) sa an eluent to give 760 mg of the title compound having the following physical characteristics:

NMR: δ = 11.20(1H, broad-s), 10.23(1H, s), 8.15(1H, d), 7.97 (1H, d), 7.27(4H, m), 2.70(2H, t), 1.3(6H, m), 0.85 (3H, t);

Mass: m/e = 345($M^+$), 298, 242, 122, 115, 91.

The next compound having the following physical characteristics was given by the same procedure described in Reference Example 16:

(a) 2-Formyl-6-nitro-4-phenylthiophenol

Starting material: 2.0 g of 2-formyl-4-phenylthiophenol (prepared in Reference Example 15(a));

Yield: 1.4 g;

TLC(benezene:ethyl acetate = 2:1): Rf = 0.45;

IR: ν = 3450, 3060, 2870, 1690, 1605, 1580, 1525, 1475, 1450, 1410, 1390, 1310, 1250, 1150, 1100, 1085, 1020, 935, 770, 740, 690 $cm^{-1}$;

NMR: δ = 11.20(1H, s), 10.09(1H, s), 8.15(1H, d), 7.92(1H, d), 7.21(5H, s);

Mass: m/e = 275($M^+$), 245, 227, 183, 171, 139.

Reference Example 17

2-[2-Ethoxycarbonyl-(1E)-vinyl]-6-nitro-4-(4-pentylphenylthio)phenol

To a solution of 760 mg of 2-formyl-6-nitro-4-(4-pentylphenylthio)phenol (prepared in Reference Example 16) dissolved in 15 ml of

chloroform, a solution of 767 mg of ethoxycarbonylvinylidene triphenylphosphorane in chloroform at room temperature. After stirring for 10 minutes at room temperature, the solution was concentrated. The residue was purified by column chromatography on silica gel using a mixture of methylene chloride and n-hexane (10:1) as an eluent to give 434 mg of the title compound having the following physical characteristics:

IR: $\nu$ = 3420, 3080, 2930, 2850, 1705, 1525, 1450, 1405, 1310, 1290, 1270, 1170, 1035, 985 $cm^{-1}$;

NMR: $\delta$ = 11.12(1H, broad-s), 7.91(1H, d), 7.73(1H, d), 7.58(1H, d), 7.10(4H, m), 6.42(1H, d), 4.20(2H, q), 2.57(2H, t), 1.30(9H, s), 0.88(3H, t);

Mass: m/e = 415($M^+$), 369, 358, 312, 123, 91.

The next compound having the following physical characteristics was given by the same procedure described in Reference Example 17:

(a) 2-[2-Ethoxycarbonyl-(1E)-vinyl]-6-nitro-4-phenylthiophenol

Starting material: 530 mg of 2-formyl-6-nitro-4-phenylthiophenol (prepared in Reference Example 16(a));

Yield: 500 mg;

TLC(benzene:ethyl acetate = 2:1): Rf = 0.68;

IR: $\nu$ = 3450, 3075, 2990, 2930, 1705, 1605, 1530, 1450, 1405, 1320, 1290, 1280, 1160, 1095, 1045, 990, 755 $cm^{-1}$;

NMR: $\delta$ = 11.25(1H, s), 8.14(1H, d), 7.88(1H, d), 7.78(1H, d), 7.35(5H ,s), 6.59(1H, d), 4.39(2H, q), 1.34(3H, t);

Mass: m/e = 345($M^+$), 299, 252, 225, 197, 152.

Example 1

2-Amino-6-[2-ethoxycarbonyl-(1E)-vinyl]-phenol hydrochloride.

To a suspension of 1.0 g of 2-[2-ethoxycarbonyl-(1E)-vinyl]-6-nitrophenol (prepared in Reference Example 2) in 20 ml of water, 10 ml of concentrated aqueous ammonia was added. To the solution, 7 g of sodium hydrosulfite in 70 ml of water was added at once and the solution was stirred at room temperature for 10 minutes. The reaction mixture was extracted with chloroform, and the extract was washed with a saturated aqueous sodium chloride, dried over anhydrous magnesium sulfate, and concentrated under reduced pressure. The residue was purified bycolumn chromatography on silica gel using a mixed solvent of methylene chloride and methanol (10:1) as an eluent to give 800 mg of the aniline compound. Obtained compound was dissolved in ethanol, added 0.5 ml of concentrated hydrochloric acid, concentrated under reduced pressure, and dried in vacuo by vacuum pump. The residue was dissolved in ethanol and treated with active carbon, concentrated under reduced pressure, and dried in vacuo by vacuum pump to give 769 mg of the title compound having the following physical characteristics:

mp : 170° - 180°C (decomposed);

TLC(methylene chloride : methanol = 10:1):Rf = 0.53;

IR : ν = 3420, 3130, 2990, 2880, 2600, 1700, 1630, 1590, 1570, 1530, 1470, 1395, 1370, 1335, 1315, 1300, 1275, 1225, 1195, 1100, 1025, 980, 860, 790 $cm^{-1}$;

NMR(methanol-$d_4$):

δ = 8.03(1H, d), 7.70(1H, dd), 7.45(1H, dd), 7.06(1H, t), 6.57(1H, d), 4.27(2H, q), 1.34(3H, t);

Mass : m/e = 207($M^+$), 161, 133, 106, 104.

The next compounds having the following physical characteristics were given from the corresponding nitro compound by the same procedure as described in Example 1:

(a) 2-Amino-4-chloro-6-[2-ethoxycarbonyl-(1E)-vinyl]phenol hydrochloride

Starting material: 450 mg of 4-chloro-2-[2-ethoxycarbonyl-(1E)-vinyl]-6-nitrophenol (prepared in Reference Example 3.).

Yield: 336 mg;

mp: 167° - 180°C (decomposed);

TLC(ethyl ether) : Rf = 0.58 (as free amine);

IR : ν = 3230, 2700, 2500, 1685, 1633, 1518, 1420, 1345, 1324, 1300, 1225, 1210, 1199, 1020, 973, 858 $cm^{-1}$;

NMR(dimethylsulfoxide-$d_6$):

δ = 7.92(1H, d), 7.66(1H, d), 7.42(1H, d), 6.68(1H, d), 4.23(1H, q), 1.28(3H, t);

Mass : m/e = 241($M^+$), 195, 167.

(b) 2-Amino-4-chloro-6-[4-ethoxycarbonyl-(1E, 3E)-butadienyl]phenol hydrochloride

Starting material: 511 mg of 4-chloro-2-[4-ethoxycarbonyl-(1E, 3E)-butadienyl]-6-nitrophenol (prepared in Reference Example 5);

Yield : 425 mg;

mp : 150° - 180°C (decomposed);

TLC(ethyl ether): Rf = 0.52;

IR : ν = 3400, 2330, 2525, 1687, 1620, 1466, 1262, 1185, 1145,

993, 865 $cm^{-1}$;

NMR(methanol-$d_4$ + acetone-$d_6$):

$\delta$ = 8.79-7.06(5H, m), 6.15(1H, d), 4.23(2H, q), 1.70(3H, t);

Mass : m/e = 267($M^+$), 238, 222, 221, 204, 193, 177, 130.

(c) Mixture of 2-amino-4-chloro-6-[3-methoxycarbonyl-(1E)-propenyl]phenol hydrochloride and 2-amino-4-chloro-6-[3-ethoxycarbonyl-(1E)-propenyl] phenol hydrochloride.

Starting material: 272 mg of 4-chloro-2-[3-methoxycarbonyl-(1E)-propenyl]-6-nitrophenol (prepared in Reference Example 6);

Yield: 265 mg;

mp : 130° - 155°C(decomposed);

TLC(ethyl ether): Rf = 0.41 (as free amine);

IR : ν = 3330, 3200, 2830, 2570, 1730, 1690, 1580, 1555, 1468, 1305, 1218, 1195, 1180, 1025, 960, 860 $cm^{-1}$;

NMR(methanol-$d_4$): $\delta$ = 7.52(1H, d), 7.35(1H, d), 6.80(1H, d), 6.30(1H, dt), 4.20(q), 3.74(s), 3.37(2H, m), 1.29(t);

Mass : m/e = 255($M^+$; ethyl compound), 241 ($M^+$; methyl compound).

(d) 2-Amino-4-[2-ethoxycarbonyl-(1E)-vinyl]-phenol hydrochloride.

Starting material: 200 mg of 4-[2-ethoxycarbonyl-(1E)-vinyl]-2-nitrophenol (prepared in Referance Example 8);

Yield: 176 mg;

mp : 185°C - 195°C (decomposed);

TLC(chloroform: ethanol = 10:1): Rf = 0.47 (as free amine);

IR : ν = 3450, 2980, 2810, 2620, 2560, 1710(shoulder), 1675,

1620, 1500, 1445, 1370, 1290(shoulder), 1280, 1240, 1190, 1130, 1095, 1040, 980, 840 $cm^{-1}$;

NMR: δ = 7.6(3H, m), 7.10(1H, d), 6.43(1H, d), 4.24(2H, q), 1.32(3H, t);

Mass: m/e = 207($M^+$), 179, 162, 145, 135.

(e) 2-Amino-6-methoxy-4-[2-methoxycarbonyl-(1E)-vinyl]phenol hydrochloride

Starting material: 300 mg of 2-methoxy-4-[2-methoxycarbonyl-(1E)-vinyl]-6-nitrophenol (prepared in Reference Example 7(a) );

Yield: 50 mg;

mp : 193° - 198°C (decomposed);

TLC(methylene chloride : methanol = 10:1): Rf = 0.59;

IR : ν = 3460(broad), 2900(broad), 2590, 2560, 1705, 1625, 1600, 1570, 1520, 1510, 1465, 1440, 1430, 1315, 1295, 1270, 1240, 1220, 1160, 1110, 1065, 1035, 985, 945, 870, 845 $cm^{-1}$;

NMR(methanol-$d_4$)

δ = 7.61(1H, d), 7.28(2H, m), 6.48(1H, d), 3.99(3H, s), 3.79(3H, s);

Mass: m/e = 223($M^+$), 208, 192, 160, 132, 120.

(f) 2-Amino-4-[2-ethoxycarbonyl-(1E)-vinyl]-6-methoxyphenol hydrochloride.

Starting material: 150 mg of 4-[2-ethoxycarbonyl-(1E)-vinyl]-2-methoxy-6-nitrophenol (prepared in Reference Example 8(a) );

Yield: 27 mg;

mp : 160° - 175°C (decomposed);

TLC(methylene chloride : methanol = 10:1): Rf= 0.66;

IR: ν = 3550, 3400(broad), 2990, 2830(broad), 2590, 2550, 1705, 1625, 1570, 1510, 1465, 1435, 1370, 1315, 1295, 1270, 1240, 1190, 1160, 1100, 1045, 985, 930, 880, 840 $cm^{-1}$;

NMR(methanol-$d_4$):

δ = 7.60(1H, d), 7.28(2H, s), 6.47(1H, d), 4.25(2H, q), 3.99(3H, s), 1.33(3H, t);

Mass: m/e = 237($M^+$), 222, 209, 196, 192, 165, 160, 132, 120.

(g) 2-Amino-4-t-butyl-6-[2-ethoxycarbonyl-(1E)-vinyl]phenol hydrochloride

Starting material: 500 mg of 4-t-butyl-2-[2-ethoxycarbonyl-(1E)-vinyl]phenol (prepared in Reference Example 11);

Yield: 412 mg;

mp: 165 - 175°C;

TLC(methylene chloride:methanol = 10:1): Rf = 0.51;

IR: ν = 3430, 2980, 2580, 1710, 1635, 1490, 1400, 1370, 1285, 1210(shoulder), 1190, 1120, 1035, 980, 865, 830, 710 $cm^{-1}$;

NMR(methanol-$d_4$): δ = 8.07(1H, d), 7.69(1H, d), 7.50(1H, d), 6.57(1H, d), 4.29(2H, q), 1.34(9H, s), 1.34(3H, t);

Mass: m/e = 263, 248, 217, 202, 174, 162, 146, 134.

(h) 2-Amino-4-[2-[4-biphenylmethoxycarbonyl]-(1E)-vinyl]-phenol

Starting material: 111 mg of 4-[2-[4-biphenylmethoxycarbonyl]-(1E)-vinyl]-2-nitrophenol (prepared in Reference Example 12);

Defference in procedure: procedure into hydrochloride was not done;

Yield: 61 mg;

mp: 205°C;

TLC(cyclohexane:ethyl acetate = 1:1): Rf = 0.26;

IR: $\nu$ = 3400, 3350, 1690, 1630, 1595, 1515, 1275, 1160 $cm^{-1}$;

NMR(methanol-$d_4$): $\delta$ = 7.2 - 7.8(10H, m), 7.04(1H, d), 6.86 (1H, dd), 6.72(1H, d), 6.34(1H, d), 5.28(2H, s);

Mass: m/e = 345($M^+$), 178, 167.

(i) 2-Amino-6-[2-ethoxycarbonyl-(1E)-vinyl]-4-(4-pentylphenylthio)phenol hydrochloride

Starting material: 336 mg of 2-[2-ethoxycarbonyl-(1E)-vinyl]-6-nitro-4-(4-pentylphenylthio)phenol (prepared in Reference Example 17);

Yield: 66 mg;

IR: $\nu$ = 3430, 3040, 2935, 2855, 2580, 1690, 1635, 1525, 1470, 1375, 1330, 1305, 1265, 1190(broad), 1115, 1020, 980, 860 $cm^{-1}$;

NMR(methanol-$d_4$): $\delta$ = 7.95(1H, d), 7.60(1H, d), 7.29(1H, d), 7.28(2H, d), 7.18(2H, d), 6.43(1H, d), 4.24(2H, q), 2.60(2H, t), 1.60(2H, m), 1.33(7H, m), 0.90(3H, t);

Mass: m/e = 385($M^+$), 339, 282, 133.

(j) 2-Amino-6-[2-ethoxycarbonyl-(1E)-vinyl]-4-phenylthiophenol hydrochloride

Starting material: 500 mg of 2-[2-ethoxycarbonyl-(1E)-vinyl]-6-nitro-4-phenylthiophenol (prepared in Reference Example 17(a));

Yield: 394 mg;

mp: 169 - 174°C (decomposed);

TLC(methylene chloride:methanol = 10:1): Rf = 0.64;

IR: ν = 3450, 3050, 2980, 2850, 2580, 1710(shoulder), 1690, 1635, 1570, 1525, 1475, 1325, 1300, 1265, 1230(shoulder), 1190, 1110, 1030, 985, 865, 755, 745, 695 $cm^{-1}$;

NMR(methanol-$d_4$): δ = 7.98(1H, d), 7.69(1H, d), 7.38(1H, d), 7.35(5H, s), 6.48(1H, d), 4.25(2H, q), 1.31(3H, t);

Mass: m/e = 315, 269, 241, 212, 133.

Example 2

2-Amino-4-methoxycarbonylmethylphenol hydrochloride.

To a solution of 450 mg of 4-methoxycarbonylmethyl-2-nitrophenol (prepared in Reference Example 7) dissolved in 10 ml of methanol, 60 mg of palladium-carbon (content: 10%) was added and air in reaction vessel was replaced to hydrogen gas, the solution was stirred for 15 hours at room temperature. The catalyst was removed from the reaction solution by filtration, and the filtrate was concentrated under reduced pressure. The residue was dissolved in methanol, added 0.2 ml of concentrated hydrochloric acid, concentrated under reduced pressure, and dried in vacuo by vacuum pump. The residue was dissolved in ethanol, treated with active

carbon, and dried in vacuo by vacuum pump to give 418 mg of the title compound having the following physical characteristics:

mp : 195° - 205°C (decomposed);

TLC (chloroform: methanol: aqueous ammonia = 60 : 6 : 1): Rf = 0.45;

IR : ν = 3240, 2950, 2860, 2600, 2580, 1705, 1645, 1610, 1580, 1535, 1515, 1455, 1440, 1370, 1335, 1295, 1275, 1210, 1155, 1145, 1005, 900, 840, 810, 700 $cm^{-1}$;

NMR(methanol-$d_4$ + deuterochloroform):

δ = 7.29(1H, d), 7.17(1H, dd), 7.00(1H, d), 3.68(3H, s), 3.60(2H, s);

Mass: m/e = 181($M^+$), 122, 94, 77.

The next compound having the following physical characteristics was given from the corresponding nitrophenol compound by the same procedure as described in Example 2:

(a) 2-Amino-4-(2-ethoxycarbonylethyl)phenol hydrochloride.

Starting material: 200 mg of 4-[2-ethoxycarbonyl-(1E)-vinyl]-2-nitrophenol (prepared in Reference Example 8);

Yield : 170 mg;

mp : 165° - 189°C (decomposed);

TLC(methylene chloride : ethanol = 10:1): Rf = 0.44 (as free amine);

IR : ν = 3450, 3070, 2980, 2570, 1735, 1635, 1560, 1510, 1470, 1450, 1370, 1350, 1290, 1255, 1210, 1125, 1040, 1025, 940, 825 $cm^{-1}$;

NMR(methanol-$d_4$)

δ = 7.22(1H, m), 7.15(1H, dd), 6.97(1H, d), 4.10(2H, q), 2.86(2H, t), 2.63(2H, t), 1.21(3H, t);

Mass : m/e = 209($M^+$), 181, 164, 135, 122, 91

(b) 2-Amino-4-t-butyl-6-(2-ethoxycarbonylethyl)phenol hydrochloride

Starting material: 500 mg of 4-t-butyl-2-[2-ethoxycarbonyl-(1E)-vinyl]phenol (prepared in Reference Example 11);

Yield: 250 mg;

mp: 115 - 120°C;

TLC(chloroform:methanol = 10:1): Rf = 0.62;

IR: $\nu$ = 3450, 2970, 2580, 1730, 1630, 1570, 1490, 1370, 1305, 1205, 1120, 1040, 880, 825, 740 $cm^{-1}$;

NMR(deuterochloroform + methanol-$d_4$):

$\delta$ = 7.28(1H, d), 7.22(1H, d), 4.15(2H, q), 2.99(2H, t), 2.69(2H, t), 1.29(9H, s), 1.23(3H, t);

Mass: m/e = 265, 219, 204, 177, 162.

Example 3

2-Amino-6-[2-carboxy-(1E)-vinyl]-phenol hydrochloride

To a solution of 100 mg of 2-amino-6-[2-ethoxycarbonyl-(1E)-vinyl]phenol hydrochloride (prepared in Example 1) in 5 ml of methanol was added 2 ml of 2N aqueous solution of sodium hydroxide and the solution was stirred at 55°C for 1 hour and 20 minutes. The solution was allowed to cool to room temperature, adjusted to pH 8 by addition of 1.5 ml of 2.4N hydrochloric acid, treated with active carbon, and concentrated under reduced pressure by vacuum pump. To the residue was added 15 ml of ethanol and 10 drops of concentrated hydrochloric acid, and the solution was refluxed and filtered at that temperature to remove insoluble sodium chloride. The filtrate was treated with active carbon, concentrated by vacuum pump to give 42 mg of the title compound having the following physical characteristics:

mp : 206° - 207°C;

TLC(ethyl acetate): Rf = 0.43;

IR : ν = 3440, 1686, 1622, 1470, 1300 $cm^{-1}$;

NMR(methanol-$d_4$):

δ = 8.02(1H, d), 7.68(1H, dd), 7.35(1H, dd), 7.05(1H, t), 6.51(1H, d);

Mass : m/e = 179, 161, 133.

The next compound having the following physical characteristics was given by the same procedure described in Example 3, with the exception of the procedure into hydrochloride:

(a) 2-Amino-6-[2-carboxy-(1E)-vinyl]-4-(4-pentylphenylthio)phenol

Starting material: 160 mg of 2-amino-6-[2-ethoxycarbonyl-(1E)-vinyl]-4-(4-pentylphenylthio)phenol hydrochloride (prepared in Example 1(i));

Yield: 80 mg;

IR: ν = 3430, 3040(shoulder), 2930, 2850, 1690, 1630, 1470, 1265, 1195, 975, 860 $cm^{-1}$;

NMR(methanol-$d_4$): δ = 7.93(1H, d), 7.56(1H, d), 7.31(1H, d), 7.29(2H, d), 7.17(2H, d), 6.37(1H, d), 2.60(2H, t), 1.61(2H, m), 1.34(4H, m), 0.90(3H, t);

Mass: m/e = 357($M^+$), 339, 313, 282, 256, 133, 123, 91.

Example 4

5 g of 2-Amino-6-[2-ethoxycarbonyl-(1E)-vinyl]phenol hydrochloride, 200 mg of cellulose magnesium gluconate (disintegrating agent), 100 mg of magnesium, stearate (lubricating agent) and 4.7 g of crystalline cellulose were admixed and punched out in a conventional manner to obtain 100 tablets containing 50 mg of the active ingredient in one tablet.

Designated States:- BE,CH/LI,DE,FR,GB,IT,LU,NL,SE.

CLAIMS

1. A 2-aminophenol derivative of the general formula:

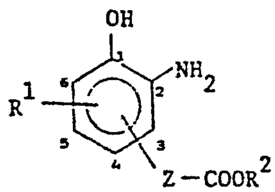

(I)

[wherein Z represents a straight alkylene or alkenylene group containing from 1 to 6 carbon atom(s), $R^1$ represents a hydrogen atom, a halogen atom, a straight or branched chain alkyl group containing from 1 to 6 carbon atom(s), a straight or branched chain alkoxy group containing from 1 to 4 carbon atom(s) or a group represented by general formula: -S-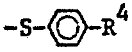-$R^4$ (wherein $R^4$ represents a hydrogen atom or a straight or branched chain alkyl group containing from 1 to 6 carbon atom(s).), $R^2$ represents a hydrogen atom, a straight or branched chain alkyl group containing from 1 to 4 carbon atom(s) or a group represented by a formula: $-CH_2-$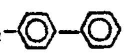, $R^1$ is attached to the 6th or 4th position of the benzene ring; when $R^1$ is attached to the 6th position of the benzene ring, Z-$COOR^2$ is attached to the 4th position of the benzene ring, and when $R^1$ is attached to the 4th position of the benzene ring, Z-$COOR^2$ is attached to the 6th position of the benzene ring, with the exception of compounds wherein $R^1$ represents a hydrogen atom and is attached to the 6th position of the benezene ring, Z represents a methylene group and $R^2$ represents a hydrogen atom or a straight or branched chain alkyl group containing from 1 to 4 carbon atom(s), and Z-$COOR^2$ is attached to the 4th position of the benzene ring], or a pharmaceutically acceptable acid addition salt thereof.

2. A compound according to claim 1 wherein $R^1$ is attached to the 6th position of the benzene ring, and Z-$COOR^2$ is attached to the 4th position of the benzene ring.

3. A compound according to claim 1 wherein $R^1$ is attached to the 4th position of the benzene ring, and Z-$COOR^2$ is attached to the 6th position of the benzene ring.

4. A compound according to claim 1 wherein Z represents an ethylene group, a vinylene group, n-propylene group or n-butadienylene group.

5. A compound according to claim 1 wherein $R^1$ represents a hydrogen atom, a chlorine atom, a methoxy group, a t-butyl group, a phenylthio group or a 4-pentylphenylthio group.

6. A compound according to claim 1 wherein $R^2$ represents a hydrogen atom, a methyl group , an ethyl group or 4-biphenylmethyl group.

7. A compound according to claim 1 which is 2-amino-6-(2-ethoxycarbonylvinyl)phenol or hydrochloride thereof.

8. A compound according to claim 1 which is 2-amino-4-chloro-6-(2-ethoxycarbonylvinyl)phenol or hydrochloride thereof.

9. A compound according to claim 1 which is 2-amino-4-chloro-6-(4-ethoxycarbonylbutadienyl)phenol or hydrochloride thereof.

10. A compound according to claim 1 which is 2-amino-4-chloro-6-(3-methoxycarbonyl-1-propenyl)phenol or hydrochloride thereof.

11. A compound according to claim 1 which is 2-amino-4-(2-ethoxycarbonylethyl)phenol or hydrochloride thereof.

12. A compound according to claim 1 which is 2-amino-4-(2-ethoxycarbonylvinyl)phenol or hydrochloride thereof.

13. A compound according to claim 1 which is 2-amino-6-methoxy-4-(2-methoxycarbonylvinyl)phenol or hydrochloride thereof.

14. A compound according to claim 1 which is 2-amino-4-(2-ethoxycarbonylvinyl)-6-methoxyphenol or hydrochloride thereof.

15. A compound according to claim 1 which is 2-amino-6-(2-carboxyvinyl)phenol or hydrochloride thereof.

16. A compound according to claim 1 which is 2-amino-4-t-butyl-6-(2-ethoxycarbonylvinyl)phenol or hydrochloride thereof.

17. A compound according to claim 1 which is 2-amino-4-t-butyl-6-(2-ethoxycarbonylethyl)phenol or hydrochloride thereof.

18. A compound according to claim 1 which is 2-amino-4-[2-(4-biphenylmethoxycarbonyl)vinyl]phenol or hydrochloride thereof.

19. A compound according to claim 1 which is 2-amino-6-(2-ethoxycarbonylvinyl)-4-(4-pentylphenylthio)phenol or hydrochloride thereof.

20. A compound according to claim 1 which is 2-amino-6-(2-carboxyvinyl)-4-(4-pentylphenylthio)phenol or hydrochloride thereof.

21. A compound accoridng to claim 1 which is 2-amino-6-(2-ethoxycarbonylvinyl)-4-phenylthiophenol or hydrochloride thereof.

22. A process for the preparation of 2-amino-phenol derivative as claimed in claim 1 and conforming to the general formula:

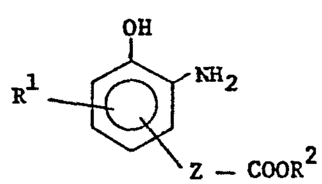

(I)

[wherein all of the symbols represent the same meaning as in claim 1 ] or a pharmaceutically acceptable acid addition salts thereof, which comprises

(i) selective reducing the nitro group, to an amino group, of a compound of the general formula:

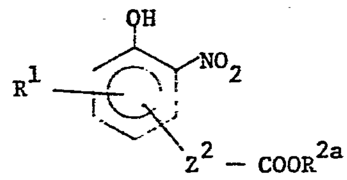

(IIa)

[wherein $Z^2$ represents a straight alkenylene group containing from 2 to 6 carbon atoms, $R^{2a}$ represents a hydrogen atom or a straight or branched chain alkyl group containing from 1 to 4 carbon atom(s), $R^1$ represents the same meaning as defined in claim 1, and the attached positions of $R^1$ and $Z^2$-$COOR^{2a}$ apply correspondingly as the ones of $R^1$ and Z-$COOR^2$ depicted in claim 1 ],

(ii) reducing the nitro group and the double bond(s) in $Z^2$ of a compound of the general formula (IIa), to an amino group and a straight alkylene group respectively, at the same time,

(iii) selective reducing the nitro group, to amino group, of a compound of the general formula:

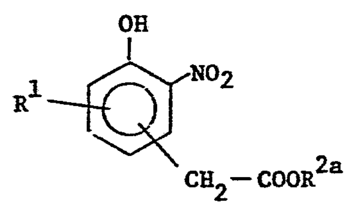

(IIb)

[wherein all of the symbols represent the same meaning as defined in claim 1 or hereinbefore depicted],

(iv) selective reducing the nitro group, to an amino group, of a compound of the general formula:

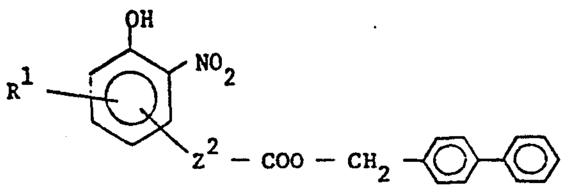

(IIc)

[wherein all of the symbols represent the same meaning as defined in claim 1 or hereinbefore depicted],

(v) esterifying of a compound of the general formula (I) wherein $R^2$ represents a hydrogen atom, or

(vi) saponifying of a compound of the general formula (I) wherein $R^2$ represents a straight or branched chain alkyl group containing from 1 to 4 carbon atom(s) or a group represented by a formula: $-CH_2-$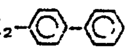 .

23. A pharmaceutical composition for the prevention and the teatment of allergic tracheal and bronchial deseases or allergic lung diseases, allergic shocks or various allergic inflammations, or for the prevention and the treatment of various inflammations induced by prostaglandins, which comprises, as active ingredient, an effective amount of at least one compound of the general formula (I) depicted in claim 1, wherein all of the symbols represent the same meaning as depicted in claim 1, or a pharmaceutically acceptable acid addition salt thereof, together with a pharmaceutical carrier or coating.

Designated States: AT

CLAIMS

1. A process for the preparation of 2-aminophenol derivative of the general formula:-

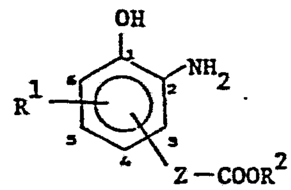

(I)

[wherein Z represents a straight alkylene or alkenylene group containing from 1 to 6 carbon atom(s), $R^1$ represents a hydrogen atom, a halogen atom, a straight or branched chain alkyl group containing from 1 to 6 carbon atom(s), a straight or branched chain alkoxy group containing from 1 to 4 carbon atom(s) or a group represented by general formula: 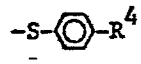 (wherein $R^4$ represents a hydrogen atom or a straight or branched chain alkyl group containing from 1 to 6 carbon atom(s).), $R^2$ represents a hydrogen atom, a straight or branched chain alkyl group containing from 1 to 4 carbon atom(s) or a group represented by a formula: 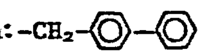, $R^1$ is attached to the 6th or 4th position of the benzene ring; when $R^1$ is attached to the 6th position of the benzene ring, Z-$COOR^2$ is attached to the 4th position of the benzene ring, and when $R^1$ is attached to the 4th position of the benzene ring, Z-$COOR^2$ is attached to the 6th position of the benzene ring, with the exception of compounds wherein $R^1$ represents a hydrogen atom and is attached to the 6th position of the benezene ring, Z represents a methylene group and $R^2$ represents a hydrogen atom or a straight or branched chain alkyl group containing from 1 to 4 carbon atom(s), and Z-$COOR^2$ is attached to the 4th position of the benzene ring ], or a pharmaceutically acceptable acid addition salt thereof, which comprises

(i) selective reducing the nitro group, to an amino group, of a compound of the general formula:

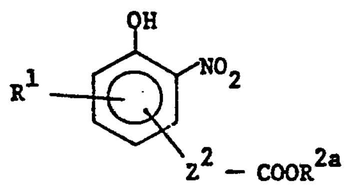

(IIa)

[wherein $Z^2$ represents a straight alkenylene group containing from 2 to 6 carbon atoms, $R^{2a}$ represents a hydrogen atom or a straight or branched chain alkyl group containing from 1 to 4 carbon atom(s), $R^1$ represents the same meaning as defined in claim 1, and the attached positions of $R^1$ and $Z^2$-$COOR^{2a}$ apply correspondingly as the ones of $R^1$ and Z-$COOR^2$]

(ii) reducing the nitro group and the double bond(s) in $Z^2$ of a compound of the general formula (IIa), to an amino group and a straight alkylene group respectively, at the same time,

(iii) selective reducing the nitro group, to amino group, of a compound of the general formula:

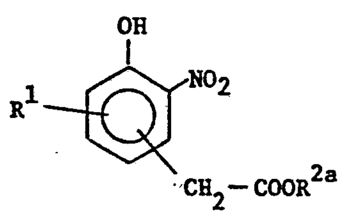

(IIb)

[wherein all of the symbols represent the same meaning as defined above ],

(iv) selective reducing the nitro group, to an amino group, of a compound of the general formula:

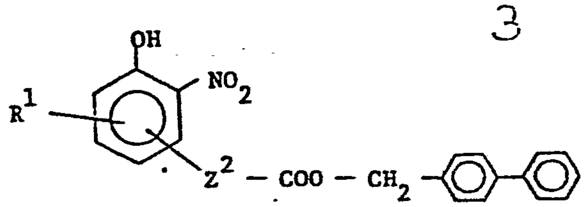

(IIc)

[wherein all of the symbols represent the same meaning as defined in claim 1 or hereinbefore depicted],

(v) esterifying of a compound of the general formula (I) wherein $R^2$ represents a hydrogen atom, or

(vi) saponifying of a compound of the general formula (I) wherein $R^2$ represents a straight or branched chain alkyl group containing from 1 to 4 carbon atom(s) or a group represented by a formula: $-CH_2-$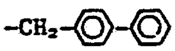 .

2. A process according to claim 1 wherein $R^1$ is attached to the 6th position of the benzene ring, and Z-$COOR^2$ is attached to the 4th position of the benzene ring.

3. A process according to claim 1 wherein $R^1$ is attached to the 4th position of the benzene ring, and Z-$COOR^2$ is attached to the 6th position of the benzene ring.

4. A process according to claim 1 wherein Z represents an ethylene group, a [illegible]lene group, n-propylene group or n-butadienylene group.

5. A process according to claim 1 wherein $R^1$ represents a hydrogen atom, a chlorine atom, a methoxy group, a t-butyl group, a phenylthio group or a 4-pentylphenylthio group.

6. A process according to claim 1 wherein $R^2$ represents a hydrogen atom, a methyl group, an ethyl group or 4-biphenylmethyl group.

7. A process according to claim 1 which is 2-amino-6-(2-ethoxycarbonylvinyl)phenol or hydrochloride thereof.

8. A process according to claim 1 which is 2-amino-4-chloro-6-(2-ethoxycarbonylvinyl)phenol or hydrochloride thereof.

9. A process according to claim 1 which is 2-amino-4-chloro-6-(4-ethoxycarbonylbutadienyl)phenol or hydrochloride thereof.

10. A process according to claim 1 which is 2-amino-4-chloro-6-(3-methoxycarbonyl-1-propenyl)phenol or hydrochloride thereof.

11. A process according to claim 1 which is 2-amino-4-(2-ethoxycarbonylethyl)phenol or hydrochloride thereof.

12. A process according to claim 1 which is 2-amino-4-(2-ethoxycarbonylvinyl)phenol or hydrochloride thereof.

13. A process according to claim 1 which is 2-amino-6-methoxy-4-(2-methoxycarbonylvinyl)phenol or hydrochloride thereof.

14. A process according to claim 1 which is 2-amino-4-(2-ethoxycarbonylvinyl)-6-methoxyphenol or hydrochloride thereof.

15. A process according to claim 1 which is 2-amino-6-(2-carboxyvinyl)phenol or hydrochloride thereof.

16. A according to claim 1 which is 2-amino-4-t-butyl-6-(2-ethoxycarbonylvinyl)phenol or hydrochloride thereof.

17. A process according to claim 1 which is 2-amino-4-t-butyl-6-(2-ethoxycarbonylethyl)phenol or hydrochloride thereof.

18. A process according to claim 1 which is 2-amino-4-[2-(4-biphenylmethoxycarbonyl)vinyl]phenol or hydrochloride thereof.

19. A process according to claim 1 which is 2-amino-6-(2-ethoxycarbonylvinyl)-4-(4-pentylphenylthio)phenol or hydrochloride thereof.

20. A process according to claim 1 which is 2-amino-6-(2-carboxyvinyl)-4-(4-pentylphenylthio)phenol or hydrochloride thereof.

21. A process accoridng to claim 1 which is 2-amino-6-(2-ethoxycarbonylvinyl)-4-phenylthiophenol or hydrochloride thereof.

European Patent Office

# EUROPEAN SEARCH REPORT

0081321

Application number

EP 82 30 6277

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| X | CH-A- 542 266 (CIBA-GEIGY)<br>* Columns 51-54: Preparative Example K: Compound 112 * | 1,2,4, 5,6,11 ,22 | C 07 C 101/72<br>C 07 C 149/43<br>A 61 K 31/215 |
| D,A | EP-A-0 031 561 (MERCK & CO.)<br>* Claims * | | |
| A | DE-A-2 449 990 (LILLY INDUSTRIES)<br>* Example 3 * & JP-A-50-70361 (Cat. D,A) | | |
| A | Chemical Abstracts vol. 87, no 9, 29 August 1977, Columbus, Ohio, USA D.W. DUNWELL et al. "Synthesis and antiinflammatory activity of some 2-aryl-6-benzoxazoleacetic acid derivatives" page 31, column 2, abstract no. 62486t, combined with C.A. CSI 87:622CS & J. Med. Chem., vol. 20, no. 6, 1977, pages 797-801<br>--- -/- | | TECHNICAL FIELDS SEARCHED (Int. Cl. 3)<br>C 07 C 101/72<br>C 07 C 149/43 |

The present search report has been drawn up for all claims

| Place of search | Date of completion of the search | Examiner |
|---|---|---|
| BERLIN | 14-02-1983 | BREW C.H. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

0081321

European Patent Office

# EUROPEAN SEARCH REPORT

Application number

EP 82 30 6277

Page 2

| DOCUMENTS CONSIDERED TO BE RELEVANT | | | CLASSIFICATION OF THE APPLICATION (Int. Cl. 3) |
|---|---|---|---|
| Category | Citation of document with indication, where appropriate, of relevant passages | Relevant to claim | |
| A | Chemical Abstracts vol. 80, no. 24, 17 June 1974, Columbus, Ohio, USA R. HAZARD et al. "Electrochemical preparation of N-hydroxyindoles. II. Controlled potential oxidation of some alpha-(o-hydroxyaminophenyl)alkenes." abstract no. 140499w, combined with CA CSI 80:3230 CS & Bull. Soc. Chim. nos. 1-2, Pt. 2), 1974, pages 121-125 --- | | |
| A | Chemical Abstract vol. 76, no. 25, 19 June 1972 Columbus, Ohio, USA C.H. BRIESKORN et al. "o-Diphenol proteins from the fruits of anise and caraway" page 339, column 1, abstract no. 152260s, combined with CA CSI 76:2988CS & Z. Lebensm.-Unters.-Forsch.,vol. 148, no. 2, 1972, pages 83-89 --- | | TECHNICAL FIELDS SEARCHED (Int. Cl. 3) |
| A | DE-A-2 526 230 (HOECHST AG) * Formula 3: example 5 * ----- | | |

The present search report has been drawn up for all claims

| Place of search | Date of completion of the search | Examiner |
|---|---|---|
| BERLIN | 14-02-1983 | BREW C.H. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document